(19) Europäisches Patentamt
European Patent Office
Office européen des brevets

(11) EP 4 177 901 A1

(12) EUROPEAN PATENT APPLICATION
published in accordance with Art. 153(4) EPC

(43) Date of publication:
10.05.2023 Bulletin 2023/19

(51) International Patent Classification (IPC):
G16H 15/00 (2018.01)     G16H 40/20 (2018.01)
G16H 10/60 (2018.01)     G16H 70/00 (2018.01)
G16H 80/00 (2018.01)     G06N 20/00 (2019.01)
G06N 3/08 (2023.01)

(21) Application number: 21833328.4

(22) Date of filing: 23.06.2021

(52) Cooperative Patent Classification (CPC):
A61B 5/7267; G16H 50/20

(86) International application number:
PCT/KR2021/007853

(87) International publication number:
WO 2022/005090 (06.01.2022 Gazette 2022/01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 03.07.2020 KR 20200082359

(71) Applicant: VUNO INC.
Seoul 06541 (KR)

(72) Inventor: SON, Jaemin
Yongin-si, Gyeonggi-do 16973 (KR)

(74) Representative: Mewburn Ellis LLP
Aurora Building
Counterslip
Bristol BS1 6BX (GB)

(54) **METHOD AND APPARATUS FOR PROVIDING DIAGNOSIS RESULT**

(57) Disclosed is a method of providing diagnostic related information for medical data, performed by one or more processors of a computing device. The method may include: calculating first diagnosis information on input medical data by using a first diagnosis network trained to output diagnosis information based on the medical data; calculating second diagnosis information on the input medical data by using a second diagnosis network trained to output diagnosis information based on a feature vector for the medical data calculated in the first diagnosis network, wherein the feature vector is calculated based on a feature map derived from a calculation process of the first diagnosis network; and generating correlation information comprising the first diagnosis information and the second diagnosis information calculated on the input medical data, based on a part of a calculation process of the first diagnosis network or a calculation process of the second diagnosis network.

Figure 3

**Description**

[Technical Field]

The present disclosure relates to a method for providing diagnostic results, and more particularly, to a method for providing a correlation between diagnostic related information and diagnosis information for medical data.

[Background Art]

An artificial neural network trains a method for extracting output data from the input data by using a fast processing rate and a large storage space, and derives the output data from new input data by applying the trained method. However, there is a problem in the art in that the artificial neural network is like a black box, so that a user cannot know, in detail, how the artificial neural network reaches an output in a given input. In other words, since an intermediate process cannot be known in detail, when the artificial neural network outputs an answer for a problem which it is difficult for the human to determine, an error of the artificial neural network cannot also be disregarded, and as a result, the human is in a dilemma regarding whether to believe in the answer. This problem is more magnified in a case where a value derived from the artificial neural network has a large impact on the user, such as medical and legal fields. Therefore, in the art, there is a continuous demand for a method or an apparatus for providing a result in which an output for an input is interpretable, i.e., transparency is high. Korean Patent Registration No "KR 10-2018-0123198" discloses a remote cervical cancer screening system based on automatic cervix reading and clinical decision support system to disclose contents that a deep learning based automatic reading algorithm reads a cervical cancer and determines whether to review the corresponding reading result.

[Disclosure]

[Technical Problem]

The present disclosure has been made in an effort to provide a correlation between diagnostic related information and diagnosis information for medical data.

[Technical Solution]

An embodiment of the present disclosure provides a method of providing diagnostic related information for medical data, performed by one or more processors of a computing device. The method may include: calculating first diagnosis information on input medical data by using a first diagnosis network trained to output diagnosis information based on the medical data; calculating second diagnosis information on the input medical data by using a second diagnosis network trained to output diagnosis information based on a feature vector for the medical data calculated in the first diagnosis network, wherein the feature vector is calculated based on a feature map derived from a calculation process of the first diagnosis network; and generating correlation information comprising the first diagnosis information and the second diagnosis information calculated on the input medical data, based on a part of a calculation process of the first diagnosis network or a calculation process of the second diagnosis network.

Alternatively, the calculating of, by the processor, first diagnosis information on input medical data by using the first diagnosis network may further include: calculating a feature vector for the input medical data in the first diagnosis network; and calculating the first diagnosis information based on the feature vector for the input medical data.

Alternatively, the feature vector may be calculated based on a result of performing a global pooling method on a feature map derived from the calculation process of the first diagnosis network.

Alternatively, the first diagnosis network may comprise two or more different sub first diagnosis networks for calculating first diagnosis information comprising different types of findings.

Alternatively, the correlation information may comprise a contribution of one or more findings comprised in the first diagnosis information to at least one disease comprised in the second diagnosis information.

Alternatively, the contribution of at least one contribution included in the first diagnosis information to at least one disease included in the second diagnosis information may be calculated based on at least one of a feature vector for the input medical data calculated in the first diagnosis network, a parameter of a final classification function comprised in the first diagnosis network, or a parameter of a final classification function comprised in the second diagnosis network.

Alternatively, the contribution of one or more findings comprised in first diagnosis information to at least one disease comprised in the second diagnosis information may be calculated based on at least one of a first partial contribution or a second partial contribution.

Alternatively, the first partial contribution may be based on odds of all findings comprised in first diagnosis information for at least one disease comprised in the second diagnosis information.

Alternatively, the second partial contribution may be based on counterfactual-odds of one or more findings comprised in the first diagnosis information for at least one disease comprised in the second diagnosis information, and the counterfactual-odds may include at least one of odds according to a probability of a situation in which one or more findings comprised in first diagnosis information for at least one disease comprised in second diagnosis information necessarily exist, an odd according to a probability of a situation in which one or more findings comprised in first diagnosis information for at least one disease comprised in second diagnosis information never exist, or an odd according to a predetermined probability of existence of one or more findings comprised in first diagnosis information for at least one disease comprised in second diagnosis information.

Alternatively, the generating correlation information may include displaying the first diagnosis information in the input medical data based at least in part on a class activation map.

Alternatively, the generating correlation information may include generating a class activation map based on at least one of a feature vector of the input medical data, a parameter of a final classification function comprised in the first diagnosis network, or a parameter of a final classification function comprised in the second diagnosis network; and displaying the first diagnosis information in the input medical data based on the class activation map.

Another embodiment of the present disclosure provides a computer program stored in a computer readable medium. When the computer program is executed in one or more processors, the computer program allows the following operations for providing diagnostic related information for medical data to be performed, and the operations may include: calculating first diagnosis information on input medical data by using a first diagnosis network trained to output diagnosis information based on the medical data; calculating second diagnosis information on the input medical data by using a second diagnosis network trained to output diagnosis information based on a feature vector for the medical data calculated in the first diagnosis network, wherein the feature vector is calculated based on a feature map derived from a calculation process of the first diagnosis network; and generating correlation information comprising the first diagnosis information and the second diagnosis information calculated on the input medical data, based on a part of the calculation process of the first diagnosis network or the calculation process of the second diagnosis network.

Another embodiment of the present disclosure provides a device for providing diagnostic related information for medical data. The method may include: a processor; and a memory in which at least one network function is stored, in which the memory may store at least one computer-executable instruction for the processor to: calculate first diagnosis information on input medical data by using a first diagnosis network trained to output diagnosis information based on medical data; calculate second diagnosis information on the input medical data by using a second diagnosis network trained to output diagnosis information based on a feature vector for the medical data calculated in the first diagnosis network, wherein the feature vector is calculated based on a feature map derived from a calculation process of the first diagnosis network; and generate correlation information comprising the first diagnosis information and the second diagnosis information calculated on the input medical data, based on a part of a calculation process of the first diagnosis network or a calculation process of the second diagnosis network.

Another embodiment of the present disclosure provides a computer program stored in a computer readable medium. When the computer program is executed by one or more processors include in a user terminal the computer program provides a user interface (UI) to display diagnostic related information for medical data, and the user interface may include: correlation information comprising first diagnosis information of input medical data and second diagnosis information of the input medical data; and the correlation information may be generated based on a part of a calculation process of first diagnosis information using a first diagnosis network and a calculation process of second diagnosis information using a second diagnosis network, and which is generated from a user terminal or a server.

Alternatively, the correlation information may comprise an odds ratio matrix comprising a contribution of one or more findings comprised in the first diagnosis information to at least one disease comprised in the second diagnosis information.

[Advantageous Effects]

According to an embodiment of the present disclosure, a correlation between diagnostic related information and diagnosis information for medical data can be provided.

[Description of Drawings]

Figure 1 is a block diagram of a computing device for providing diagnostic related information for medical data according to an embodiment of the present disclosure.
Figure 2 is a schematic view illustrating a network function used for providing the diagnostic related information for the medical data according to an embodiment of the present disclosure.

Figure 3 is a flowchart for a training process of a global model for outputting first diagnosis information and second diagnosis information from input medical data according to an embodiment of the present disclosure for providing diagnostic related information for medical data.

Figure 4 is a flowchart for training a second diagnosis network according to an embodiment of the present disclosure for providing diagnostic related information for medical data.

Figure 5 is a diagram for visualizing correlation information including first diagnosis information and second diagnosis information for input medical data according to an embodiment of the present disclosure for providing diagnostic related information for medical data.

Figure 6 is a diagram for a user interface displaying correlation information including first diagnosis information and second diagnosis information for input medical data according to an embodiment of the present disclosure for providing diagnostic related information for medical data.

Figure 7 is a simple and normal schematic view of a computing environment in which the embodiments of the present disclosure may be implemented.

[Mode for Invention]

Various embodiments are described with reference to the drawings. In the present specification, various descriptions are presented for understanding the present disclosure. However, it is obvious that the embodiments may be carried out even without a particular description.

Terms, "component," "module," "system," and the like used in the present specification indicate a computer-related entity, hardware, firmware, software, a combination of software and hardware, or execution of software. For example, a component may be a procedure executed in a processor, a processor, an object, an execution thread, a program, and/or a computer, but is not limited thereto. For example, both an application executed in a computing device and a computing device may be components. One or more components may reside within a processor and/or an execution thread. One component may be localized within one computer. One component may be distributed between two or more computers. Further, the components may be executed by various computer readable media having various data structures stored therein. For example, components may communicate through local and/or remote processing according to a signal (for example, data transmitted to another system through a network, such as the Internet, through data and/or a signal from one component interacting with another component in a local system and a distributed system) having one or more data packets.

A term "or" intends to mean comprehensive "or" not exclusive "or." That is, unless otherwise specified or when it is unclear in context, "X uses A or B" intends to mean one of the natural comprehensive substitutions. That is, when X uses A, X uses B, or X uses both A and B, "X uses A or B" may be applied to any one among the cases. Further, a term "and/or" used in the present specification shall be understood to designate and include all of the possible combinations of one or more items among the listed relevant items.

It should be understood that a term "include" and/or "including" means that a corresponding characteristic and/or a constituent element exists. Further, a term "include" and/or "including" means that a corresponding characteristic and/or a constituent element exists, but it shall be understood that the existence or an addition of one or more other characteristics, constituent elements, and/or a group thereof is not excluded. Further, unless otherwise specified or when it is unclear in context that a single form is indicated in context, the singular shall be construed to generally mean "one or more" in the present specification and the claims.

The term "at least one of A and B" should be interpreted to mean "the case including only A," "the case including only B," and "the case where A and B are combined."

Those skilled in the art shall recognize that the various illustrative logical blocks, configurations, modules, circuits, means, logic, and algorithm operations described in relation to the embodiments additionally disclosed herein may be implemented by electronic hardware, computer software, or in a combination of electronic hardware and computer software. In order to clearly exemplify interchangeability of hardware and software, the various illustrative components, blocks, configurations, means, logic, modules, circuits, and operations have been generally described above in the functional aspects thereof. Whether the functionality is implemented as hardware or software depends on a specific application or design restraints given to the general system. Those skilled in the art may implement the functionality described by various methods for each of the specific applications. However, it shall not be construed that the determinations of the implementation deviate from the range of the contents of the present disclosure.

The description about the presented embodiments is provided so as for those skilled in the art to use or carry out the present disclosure. Various modifications of the embodiments will be apparent to those skilled in the art. General principles defined herein may be applied to other embodiments without departing from the scope of the present disclosure. Therefore, the present disclosure is not limited to the embodiments presented herein. The present disclosure shall be interpreted within the broadest meaning range consistent to the principles and new characteristics presented herein.

The term "image" or "image data" used throughout the detailed description and claims of the present disclosure refers to multi-dimensional data constituted by discrete image elements (e.g., pixels in a 2D image), and in other words, refers to an object which may be seen with an eye (e.g., displayed on a video screen) or a digital representation of the object (such as a file corresponding to a pixel output of CT, MRI detector, etc.).

For example, the "image" may be computed tomography (CT), magnetic resonance imaging (MRI), a fungus image, ultrasonic waves, or a medical image of a subject collected by any other medical imaging system known in the technical field of the present disclosure. The image may not particularly be provided in a medical context, and may be provided in a non-medical context, and may be for example, a security search X-ray imaging.

Throughout the detailed description and claims of the present disclosure, a 'Digital Imaging and Communications in Medicine (DICOM)' standard is a term which collectively refers to several standards used for digital image representation and communication in a medical device, so that the DICOM standard is announced by the Federation Committee, constituted in the American College Radiology and the National Electrical Manufacturers Association (NEMA).

Further, throughout the detailed description and claims of the present disclosure, a Picture Archiving and Communication System (PACS)' is a term that refers to a system for performing storing, processing, and transmitting according to the DICOM standard, and medical images acquired by using digital medical image equipment such as X-ray, CT, and MRI may be stored in a DICOM format and transmitted to terminals inside or outside a hospital through a network, and additionally include a reading result and a medical chart.

In the specification of the present disclosure, "first diagnosis information" and "second diagnosis information" may mean, among intermediate data which a network function calculates for a final diagnosis from input medical data, the type of diagnosis information called according to the calculated or output order. A processor 110 may output (or generate) the first diagnosis information from the input medical data through a first diagnosis network included in a global model. The processor 110 may output (or generate) second diagnosis information through a second diagnosis network included in the global model based on at least some of the first diagnosis information. More specifically, the processor 110 may output (or generate) second diagnosis information through a second diagnosis network based on a feature vector for calculating the first diagnosis information. The first diagnosis information may include intermediate diagnosis information for generating a final diagnostic result for input medical data. The second diagnosis information may include the final diagnostic result for the input medical data.

In this specification, the first diagnosis information may include information on at least one finding. Throughout this specification, the information on the finding and finding information may be inter-exchanged and used as the same meaning. The finding information may include an existence probability, a type, a location, etc., of a specific finding which exists in input data. In this specification, the second diagnosis information may include information on at least one disease. Throughout this specification, the information on the disease and disease information may be inter-exchanged and used as the same meaning. The disease information may include an existence probability, a type, etc., of a specific disease which exists in the input data. The first diagnosis information may include data related to a clinical finding for the input medical data. Throughout this specification, the finding information may be used as a meaning including data, a lesion, or information on a region of the corresponding lesion which becomes a basis of determination of a final disease. The second diagnosis information may include determination for existence of the disease. One or more finding information may be involved in determining existence of one disease included in the second diagnosis information. One finding information may be involved in determining existence of two or more different diseases included in the second diagnosis information.

In general, a specific disease is diagnosed based on existence of at least one specific finding. For example, in diagnosing the disease of lung cancer, a first finding that a rale is auscultated during inhalation at portions below both lungs and a second finding that a reticular opacity appears at a portion below the lung in thoracic CT are aggregated to make a final diagnosis. As another example, in diagnosing a disease such as the glaucoma, a first finding according to a damage degree of the retinal nerve fiber layer and a second finding according to the vessel change of the optic nerve nipple periphery and the macula portion may be involved. As described above, throughout this specification, the finding information on at least one finding may be appreciated as an independent variable which causes the disease and the second diagnosis information including an existence probability of at least one disease diagnosed based on the information on the at least one finding may be appreciated as a dependence variable according to the independent variable. It will be apparent to those skilled in the art that the type and finding of disease described above are just examples not to limit the present disclosure, and the embodiments of the disease and the finding need not be limited in the present disclosure for quantifying which effect the change of the independent variable exerts on the result of the dependent variable.

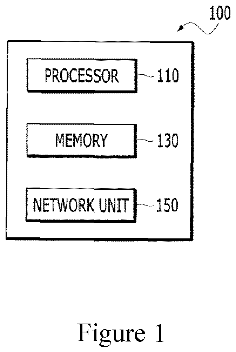

Figure 1 is a block diagram of a computing device for providing diagnostic related information for medical data according to an embodiment of the present disclosure.

A configuration of the computing device 100 illustrated in Figure 1 is only an example shown through simplification. In an embodiment of the present disclosure, the computing device 100 may include other components for performing a computing environment of the computing device 100 and only some of the disclosed components may constitute the computing device 100.

The computing device 100 may include a processor 110, a memory 130, and a network unit 150.

The processor 110 may be constituted by one or more cores and may include processors for data analysis and deep learning, which include a central processing unit (CPU), a general purpose graphics processing unit (GPGPU), a tensor processing unit (TPU), and the like of the computing device. The processor 110 may read a computer program stored in the memory 130 to perform data processing for machine learning according to an embodiment of the present disclosure. According to an embodiment of the present disclosure, the processor 110 may perform a calculation for learning the neural network. The processor 110 may perform calculations for learning the neural network, which include processing of input data for learning in deep learning (DL), extracting a feature in the input data, calculating an error, updating a weight of the neural network using backpropagation, and the like. At least one of the CPU, GPGPU, and TPU of the processor 110 may process learning of a network function. For example, both the CPU and the GPGPU may process the learning of the network function and data classification using the network function. Further, in an embodiment of the present disclosure, processors of a plurality of computing devices may be used together to process the learning of the network function and the data classification using the network function. Further, the computer program executed in the computing device according to an embodiment of the present disclosure may be a CPU, GPGPU, or TPU executable program.

According to an embodiment of the present disclosure, the memory 130 may store any type of information generated or determined by the processor 110 or any type of information received by the network unit 150.

According to an embodiment of the present disclosure, the memory 130 may include at least one type of storage medium of a flash memory type storage medium, a hard disk type storage medium, a multimedia card micro type storage medium, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. The computing device 100 may operate in connection with a web storage performing a storing function of the memory 130 on the Internet. The description of the memory is just an example and the present disclosure is not limited thereto.

The network unit 150 according to an embodiment of the present disclosure may use various wired communication systems such as public switched telephone network (PSTN), x digital subscriber line (xDSL), rate adaptive DSL (RADSL), multi rate DSL (MDSL), very high speed DSL (VDSL), universal asymmetric DSL (UADSL), high bit rate DSL (HDSL), and local area network (LAN).

Further, the network unit 150 presented in this specification may use various wireless communication systems such as code division multi access (CDMA), time division multi access (TDMA), frequency division multi access (FDMA), orthogonal frequency division multi access (OFDMA), single carrier-FDMA (SC-FDMA), and other systems.

In the present disclosure, the network unit 150 may be configured regardless of communication modes such as wired and wireless modes and constituted by various communication networks including a personal area network (PAN), a wide area network (WAN), and the like. Further, the network may be known World Wide Web (WWW) and may adopt a wireless transmission technology used for short-distance communication, such as infrared data association (IrDA) or Bluetooth.

The techniques described in this specification may also be used in other networks in addition to the aforementioned networks.

In an embodiment of the present disclosure, the processor 110 may calculate first diagnosis information on input medical data by using a first diagnosis network trained to output first diagnosis information based on medical data in order to provide diagnostic related information.

In the specification of the present disclosure, the medical data may include at least one of image data, voice data, and time-series data. That is, any type of data through which a person who works in a medical business or a device for diagnosis may determine existence of a disease in data may be included in the medical data according to the present disclosure. The image data includes all image data outputted by photographing or measuring a diseased area of a patient through inspection equipment, and converting the photographed or measured diseased area into an electrical signal. The image data may include image data constituting each frame of a moving picture in a moving picture continuously photographed according to the time from a medical image photographing device. For example, the image data includes ultrasonic inspection image data, image data by an MRI device, CT tomography image data, X-ray photographing image data, and the like. Further, when the voice data is converted into the electrical signal and output as a graph-form image or the time-series data is represented as visualized data such as a graph, etc., the corresponding image or data may be included in the image data. The above-described example of the medical data is just one example, and does not limit the present disclosure.

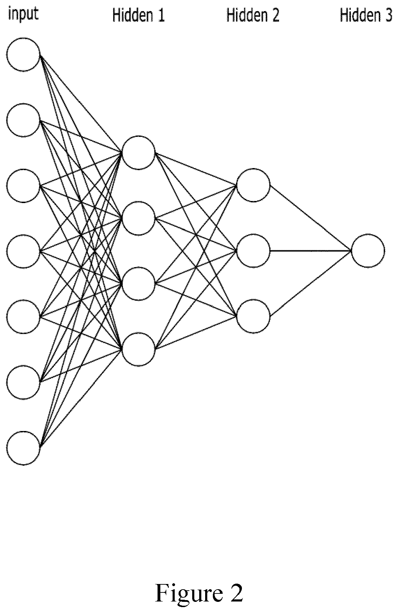

Figure 2 is a schematic view illustrating a network function used for providing the diagnostic related information for the medical data according to an embodiment of the present disclosure. Throughout the present specification, a computation model, the neural network, a network function, and the neural network may be used as the same meaning. The neural network may be generally constituted by an aggregate of calculation units which are mutually connected to each other, which may be called nodes. The nodes may also be called neurons. The neural network is configured to include one or more nodes. The nodes (alternatively, neurons) constituting the neural networks may be connected to each other by one or more links.

In the neural network, one or more nodes connected through the link may relatively form the relationship between an input node and an output node. Concepts of the input node and the output node are relative and a predetermined (or selected) node which has the output node relationship with respect to one node may have the input node relationship in the relationship with another node and vice versa. As described above, the relationship of the input node to the output node may be generated based on the link. One or more output nodes may be connected to one input node through the link and vice versa.

In the relationship of the input node and the output node connected through one link, a value of the output node may be determined based on data input in the input node. Here, a node connecting the input node and the output node to each other may have a weight. The weight may be variable and the weight is variable by a user or an algorithm in order for the neural network to perform a desired function. For example, when one or more input nodes are mutually connected to one output node by the respective links, the output node may determine an output node value based on values input in the input nodes connected with the output node and the weights set in the links corresponding to the respective input nodes.

As described above, in the neural network, one or more nodes are connected to each other through one or more links to form a relationship of the input node and output node in the neural network. A characteristic of the neural network may be determined according to the number of nodes, the number of links, correlations between the nodes and the links, and values of the weights granted to the respective links in the neural network. For example, when the same number of nodes and links exist and there are two neural networks in which the weight values of the links are different from each other, it may be recognized that two neural networks are different from each other.

The neural network may be configured to include one or more nodes. Some of the nodes constituting the neural network may constitute one layer based on the distances from the initial input node. For example, a set of nodes of which distance from the initial input node is n may constitute n layers. The distance from the initial input node may be defined by the minimum number of links which should be passed through for reaching the corresponding node from the initial input node. However, definition of the layer is predetermined (or selected) for description and the order of the layer in the neural network may be defined by a method different from the aforementioned method. For example, the layers of the nodes may be defined by the distance from a final output node.

The initial input node may mean one or more nodes in which data is directly input without passing through the links in the relationships with other nodes among the nodes in the neural network. Alternatively, in the neural network, in the relationship between the nodes based on the link, the initial input node may mean nodes which other input nodes connected through the links do not have. Similarly thereto, the final output node may mean one or more nodes which do not have the output node in the relationship with other nodes among the nodes in the neural network. Further, a hidden node may mean not the initial input node and the final output node but the nodes constituting the neural network. In the neural network according to an embodiment of the present disclosure, the number of nodes of the input layer may be the same as the number of nodes of the output layer, and the neural network may be a neural network of a type in which the number of nodes decreases and then, increases again from the input layer to the hidden layer. Further, in the neural network according to another embodiment of the present disclosure, the number of nodes of the input layer may be smaller than the number of nodes of the output layer, and the neural network may be a neural network of a type in which the number of nodes decreases from the input layer to the hidden layer. Further, in the neural network according to yet another embodiment of the present disclosure, the number of nodes of the input layer may be larger than the number of nodes of the output layer, and the neural network may be a neural network of a type in which the number of nodes increases from the input layer to the hidden layer. The neural network according to still yet another embodiment of the present disclosure may be a neural network of a type in which the neural networks are combined.

A deep neural network (DNN) may refer to a neural network that includes a plurality of hidden layers in addition to the input and output layers. When the deep neural network is used, the latent structures of data may be determined. That is, potential structures of photos, text, video, voice, and music (e.g., what objects are in the picture, what the content and feelings of the text are, what the content and feelings of the voice are) may be determined. The deep neural network may include a convolutional neural network, a recurrent neural network (RNN), an auto encoder, generative adversarial networks (GAN), a restricted Boltzmann machine (RBM), a deep belief network (DBN), a Q network, a U network, a Siam network, and the like. The description of the deep neural network described above is just an example and the present disclosure is not limited thereto.

In an embodiment of the present disclosure, the network function may include the auto encoder. The auto encoder may be a kind of artificial neural network for outputting output data similar to input data. The auto encoder may include at least one hidden layer and odd hidden layers may be disposed between the input and output layers. The number of nodes in each layer may be reduced from the number of nodes in the input layer to an intermediate layer called a bottleneck layer (encoding), and then expanded symmetrical to reduction to the output layer (symmetrical to the input layer) in the bottleneck layer. In this case, in the example of Figure 2, it is illustrated that the dimension reduction layer and the dimension reconstruction layer are symmetric, but the present disclosure is not limited thereto and the nodes of the dimension reduction layer and the dimension reconstruction layer may be symmetric or not. The auto encoder may perform non-linear dimensional reduction. The number of input and output layers may correspond to the number of sensors remaining after preprocessing the input data. The auto encoder structure may have a structure in which the number of nodes in the hidden layer included in the encoder decreases as a distance from the input layer increases. When the number of nodes in the bottleneck layer (a layer having a smallest number of nodes positioned between an encoder and a decoder) is too small, a sufficient amount of information may not be delivered, and as a result, the number of nodes in the bottleneck layer may be maintained to be a specific number or more (e.g., half of the input layers or more).

The neural network may be learned in at least one scheme of supervised learning, unsupervised learning, and semi supervised learning. Learning of the neural network is to minimize errors in output. The learning of the neural network is a process of repeatedly inputting learning data into the neural network and calculating the output of the neural network for the learning data and the error of a target and back-propagating the errors of the neural network from the output layer of the neural network toward the input layer in a direction to reduce the errors to update the weight of each node of the neural network. In the case of the supervised learning, the learning data labeled with a correct answer is used for each learning data (i.e., the labeled learning data) and in the case of the unsupervised learning, the correct answer may not be labeled in each learning data. That is, for example, the learning data in the case of the supervised learning related to the data classification may be data in which category is labeled in each learning data. The labeled learning data is input to the neural network, and the error may be calculated by comparing the output (category) of the neural network with the label of the learning data. As another example, in the case of the unsupervised learning related to the data classification, the learning data as the input is compared with the output of the neural network to calculate the error. The calculated error is back-propagated in a reverse direction (i.e., a direction from the output layer toward the input layer) in the neural network and connection weights of respective nodes of each layer of the neural network may be updated according to the back propagation. A variation amount of the updated connection weight of each node may be determined according to a learning rate. Calculation of the neural network for the input data and the back-propagation of the error may constitute a learning cycle (epoch). The learning rate may be applied differently according to the number of repetition times of the learning cycle of the neural network. For example, in an initial stage of the learning of the neural network, the neural network ensures a certain level of performance quickly by using a high learning rate, thereby increasing efficiency and uses a low learning rate in a latter stage of the learning, thereby increasing accuracy.

In learning of the neural network, the learning data may be generally a subset of actual data (i.e., data to be processed using the learned neural network) of actual data, and as a result, there may be a learning cycle in which errors for the learning data decrease, but the errors for the actual data increase. Overfitting is a phenomenon in which the errors for the actual data increase due to excessive learning of the learning data. For example, a phenomenon in which the neural network that learns a cat by showing a yellow cat sees a cat other than the yellow cat and does not recognize the corresponding cat as the cat may be a kind of overfitting. The overfitting may act as a cause which increases the error of the machine learning algorithm. Various optimization methods may be used in order to prevent the overfitting. In order to prevent the overfitting, a method such as increasing the learning data, regularization, dropout of omitting a part of the node of the network in the process of learning, etc., may be applied.

For example, the first diagnosis network is formed in a single model structure to generate first diagnosis information including information on at least one finding for the input medical data. The first diagnosis network may output a plurality of different first diagnosis information each including information on a first finding or a second finding from the input medical data. The first diagnosis network may extract at least one feature map from the input medical data, calculate the feature vector based on the feature map, and then calculate first diagnosis information including at least one finding information based on the feature vector. A learning method and an inference method of the first diagnosis network will be described below in detail.

Each of at least one finding information included in the first diagnosis information may include the type of finding which exists in the input medical data. The finding information may include at least one information of an existence probability (confidence score) of the finding, a location of the finding, and a region of the finding.

The type of finding included in the finding information may be a classification result through a deep learning method. The classification includes both one to one classification for the input medical data and the finding or one to many classification. As an embodiment, the classification may be the one to many classification for matching the input medical data to the specific finding. That is, when the number of findings which may be classified exists as N, the processor 110 may classify input data to be classified into any one class among N classification classes. The classification class may include a class meaning that there is no finding. A result of the classification may include the type of finding and the existence probability (confidence score) of the finding. As another embodiment, the classification may be the one to one classification for determining existence of all types of findings learned for the input medical data. That is, when the number of findings which may be classified by using the first diagnosis network exists as N, the processor 110 may calculate individual existence probabilities for all findings or whether all findings exist for the input medical data. In this case, the first diagnosis information for the input medical data may include information on a maximum of N findings. The processor 110 may calculate each of an existence probability of a first finding, an existence probability of a second finding, ..., an existence probability of an N-th finding for the input medical data. The classification may be comprised of multi-dimensional classifications by setting a classification interval according to the existence probability. The classification may also be binary classification through comparison of a predetermined (or selected) threshold and an existence probability. For the classification, the first diagnosis network may include multiple sub first diagnosis networks.

The location of the finding included in the finding information may be a result of detection by the deep learning based first diagnosis network. That is, when the processor 110 determines that a specific finding exists among the types of finding learned from the input medical data, the processor 110 may display a corresponding portion. The existence determination may be performed based on a probability value derived by the network function and a predetermined (or selected) threshold. The corresponding portion may be displayed by a predetermined (or selected) shape bounding box.

The information on the region of the finding included in the finding information may be a learning result of the deep learning based first diagnosis network or a result of segmentation through a network. That is, when the processor 110 outputs the finding information in the input medical data, the processor 110 displays a pixel including a lesion region regarding the corresponding finding differently from another pixel to output the finding information.

In an embodiment of the present disclosure, the processor 110 may calculate a feature vector for the input medical data in the first diagnosis network and calculate the first diagnosis information based on the feature vector for the input medical data. That is, the processor 110 may calculate a feature vector based on the feature map derived from a calculation process of the first diagnosis network and output the first diagnosis information based thereon. A calculation result of the first diagnosis network may include the feature map and the feature vector for the input medical data derived from each layer during an internal calculation process through the first diagnosis network.

The processor 110 may calculate the feature vector for the input medical data at least partially based on the feature map generated during an internal calculation process as a first diagnosis network calculation result. The feature map which becomes a basis of the generation of the feature vector may be one of at least one internal output data output from each layer included in the first diagnosis network. Each layer included in the first diagnosis network includes a convolutional layer, a pooling layer, a fully connected layer, and the like which exist in the first diagnosis network. The above-described example of the layer is just an example, and includes all layers inside a first diagnosis network structure for generating the finding information from the medical data without a limit.

The closer to a last layer in the first diagnosis network, internal output data of which abstraction is more proceeded from the input medical data may be calculated. Further, the closer to the last layer, the internal output data may include only information required for generating the final finding information. Accordingly, the feature vector for the input medical data may be generated based on a last derived feature map among one or more internal output data which exist in the first diagnosis network. The feature map which becomes the basis of the generation of the feature vector for the input medical data may be a feature map input into the fully connected layer for final classification of the finding from the input medical data in the first diagnosis network. However, the closer to the last layer in the first diagnosis network, data loss may occur from the input medical data, and as a result, the feature vector for the input medical data may also be generated based on a feature map at a predetermined (or selected) location among one or more internal output data which exist in the first diagnosis network.

At least one feature map included in the calculation result of the first diagnosis network may be expressed as an array having sizes of a predetermined (or selected) channel number C, a predetermined (or selected) height H, and a predetermined (or selected) width W. When the feature map is expressed as the array, main information including position information, etc., may not be lost for initial input data during an inference process. In this case, the feature vector generated based on the feature map may also be an array having a predetermined (or selected) channel number C', a predetermined (or selected) height H', and a predetermined (or selected) width W'. In this case, each of the predetermined (or selected) height H' and the predetermined (or selected) width W' of the feature vector may be 1 and a dimension of the feature vector may be equal to the size of the predetermined (or selected) channel number C'.

The processor 110 may perform a data flatten task by using a flatten layer in order to generate the feature vector based on the feature map. In this case, the processor 110 may perform a task of listing internal elements in the feature map in line according to a predetermined (or selected) order and converting the listed internal elements into a 1-dimensional array in order to input the feature map which is a 3D array data type into a fully connected neural network which exists last in the artificial neural network. That is, 3D data is converted into 1D data. The processor 10 may use the converted 1D data as the feature vector. As another embodiment, the processor 110 may generate the feature vector for the input medical data based on a feature map of which dimension is reduced after performing a separate pooling method for the feature map in order to generate the feature vector based on the feature map. Specifically, the processor 110 may extract the feature vector for the input medical data after reducing the dimension from the feature map by using the global pooling method.

According to an embodiment of the present disclosure, the processor 110 may calculate the feature vector for at least one input medical data based on a result of performing the global pooling method in the feature map included in the calculation of the first diagnosis network.

The global pooling method includes a global average pooling method and a global maximum pooling method. The global pooling method means a method for generating one representative value representing a channel from multiple parameter values which exist in respective channels. The global average pooling may convert an average value of parameter values which exist in one channel into a representative value. The global maximum pooling may convert a maximum value of the parameter values which exist in one channel into the representative value. For example, when a size of a feature map which is derived last among feature maps which exist as the intermediate data in the first diagnosis network is 512 (C) x 5 (H) x 5 (W), the feature vector derived when the global pooling to the feature map is applied may have a size of 512 (C') x 1 (H') x 1 (W'). The processor 110 may extract the feature vector for the input medical data by using the global pooling method and output the first diagnosis information based thereon. The processor 110 may calculate a meaningful value for each channel from each feature map by using the global pooling method. The processor 110 may skip a data flatten task for connecting a feature map of an array type having a 3D size to the fully connected (FC) layer by using the global pooling method. The processor 110 reduces the dimension of the feature map through the global pooling method to reduce the number of parameters including a weight, a bias, and the like for outputting the first diagnosis information and save a memory space for storing the parameters. For example, if the size of the feature map is 5 x 5 x 5, when the feature vector is extracted by the data flatten task, the size of the feature vector becomes 125. On the contrary, when the feature vector is generated through the global pooling method, the size of the feature vector becomes 5. This has an effect of being capable of saving the memory space and preventing a data overfitting problem of the network function for the input data.

According to an embodiment of the present disclosure, the processor 110 may include two or more different sub first diagnosis networks that calculate first diagnosis information including different types of findings. For example, a sub first diagnosis network for finding A may be learned through a learning data set labeled with finding A and a sub first diagnosis network for finding B may be learned through a learning data set labeled with finding B. Each sub first diagnosis network may perform binary classification for whether there is the corresponding finding, classification for the location and the region of the finding, etc. The number of findings which each sub first diagnosis network may calculate is one or more, and there is no limit in upper limit number.

The sub first diagnosis network may exist as large as the number of findings which the processor 110 intends to check in the input medical data through the first diagnosis network. In this case, each first diagnosis information calculated by each sub first diagnosis network may include an existence probability of one finding. In another embodiment, the sub first diagnosis network may exist in a number smaller than the number of findings. In this case, the first diagnosis information calculated by each sub first diagnosis network may include information on two or more findings. Further, the sub first diagnosis network exits in a number larger than the number of findings, and as a result, at least one sub first diagnosis network is ensembled to generate first diagnosis information including one finding information. The number of sub first diagnosis networks is just an example, and includes all numbers which may be selected by a user by comparing efficiency according to the number of sub networks and accuracy of the first diagnosis information.

The first diagnosis network may include a universal feature extraction network in order to extract a universal feature or perform a preprocessing operation. The universal feature extraction network may extract a general feature which exists regardless of a specific finding in the input medical data and then perform the preprocessing operation for the input data. The processing operation of extracting the general feature by the universal feature extraction network may include, for example, an operation of cropping a region including a blood vessel when sub first diagnosis networks for all of one or more findings generate the first diagnosis information only for the region including the blood vessel in the input data. As another example, the preprocessing operation of extracting the general feature by the universal feature extraction network may include an operation of removing a region in which the sub first diagnosis network is not commonly interested from the input data by the sub first diagnosis networks for all of one or more findings, etc. The universal feature extraction network may remove noise which exists in the input medical data. Further, the universal feature extraction network may map the input medical data to a potential space prior to inputting initial input medical data into sub first diagnosis networks for individual findings. For example, when a size of input image data having an RGB value is 512 x 512 x 3, the universal feature extraction network may map the input image data to a multi-dimensional array on a potential space having a size of 512 x 512 x 1024 in order to extract universal features including a thickness of a line, a type of line, a texture of an image, and the like. When the processor 110 extracts the universal features through the universal feature extraction network, each of all sub first diagnosis networks need not perform the same task for an initial input, and individual sub networks concentrate on feature extraction required for each finding to concentrate computing resources, thereby increasing a capability of an entire network. The processor 110 connects sub first diagnosis networks corresponding to findings, respectively to the above-described universal feature extraction network in parallel to extract feature vectors for one or more different input medical data for each finding and output first diagnosis including each finding information. The sub first diagnosis networks corresponding to the respective findings may exist as large as the number of findings labeled to learning data according to data to be learned. An example of the structure of the first diagnosis network is just an example and does not limit the present disclosure. In the present disclosure, the first diagnosis network includes all structures capable of finally calculating the first diagnosis information including at least one finding information for the input medical data without a limit.

In an embodiment of the present disclosure, the processor 110 may calculate second diagnosis information for input medical data by using a second diagnosis network trained to output the second diagnosis information based on a feature vector for the input medical data calculated in the first diagnosis network. The second diagnosis information may include a final diagnostic result for the input medical data. The second diagnosis information may include the type of disease, the existence of the disease, the probability for the existence of the disease, and the like for the input medical data.

In an embodiment of the present disclosure, the processor 110 may obtain a medical data set (i.e., a first learning data set) labeled with at least finding information and a medical data set (i.e., a second learning data set) labeled with at least one disease information in order to provide the diagnostic related information for the medical data. In the present disclosure, the medical data includes all types of medical data which may become a ground or an assistance to identify the location of the lesion or diagnose the disease.

The processor 110 may obtain the two types of learning data sets from an internal memory 130, an external memory, or an external entity of the computing device. The first learning data set the second learning data set obtained by the processor 110 may include data labeled with at least one finding information and at least one disease information for at least one same medical data. When obtaining the learning data set from the data set labeled with the finding information and the disease information for the at least one medical data, the processor 110 may obtain first medical data labeled with the finding information as the first learning data set and obtain first medical data labeled with the disease information as the second learning data set. The processor 110 may also obtain the first learning data set and the second learning data set labeled with the finding information and the disease information for each of the first medical data and the second medical data including at least one different medical data.

In an embodiment of the present disclosure, the processor 110 may train the first diagnosis network so as to output the first diagnosis information including at least one finding information by inputting the medical data included in the first learning data. That is, the processor 110 may train the deep learning based first diagnosis network to output the finding information for at least one finding by inputting the first learning data. The finding information according to an embodiment of the present disclosure may include information which may become a ground for medical determination for the corresponding learning data, and include, for example, at least one information on the existence of the finding for the corresponding learning data, information on the existence location of the finding, or information on the region of the finding.

In an embodiment of the present disclosure, the processor 110 may calculate the feature vector for the input medical data by inputting the medical data included in the second learning data into the first diagnosis network which is pretrained. Further, the processor 110 may train parameters included in the second diagnosis network so as to output the second diagnosis information including the diagnosis probability of at least one disease by inputting the feature vector for the input medical data into the second diagnosis network.

The processor 110 may train the second diagnosis network to calculate the feature vector for the input medical data related to at least one finding by inputting the medical data included in the second learning data into the deep learning based first diagnosis network and output the second diagnosis information based on the calculated feature vector. That is, when training the second diagnosis network, the first diagnosis network has already been trained to be provided to training of the second diagnosis network while internal parameters and models are fixed.

Hereinafter, a method for updating or training internal parameters included in the first diagnosis network and the second diagnosis network by the processor 110 will be described in detail through an equation. The first diagnosis network may calculate the existence probability of at least one finding included in the first diagnosis information which is the final output according to Equation 1.

[Equation 1]

$$\hat{y}_f(x) = \sigma\left(w_f^T g_f(x) + b_f\right) \quad (f \in \{f_1, f_2, \ldots, f_F\})$$

x represents the input medical data and $\hat{y}_f(x)$ represents an existence probability of a specific finding f included in the first diagnosis information which is the output of the first diagnosis network for the input medical data. f as an element of a finding set expressed as $\{f_1, f_2, \ldots, f_F\}$ means an individual finding. $g_f(x) \in R^C$ represents the feature vector for the input medical data calculated from the input data input into the first diagnosis network. $w_f$ represents an individual finding weight vector and $b_f$ represents an individual finding bias value. $\sigma$ represents an activation function.

The feature vector $g_f(x)$ for the input medical data may exist every specific finding f. The feature vector for the input medical data may be a feature vector for calculating existence probabilities of at least two findings. In the embodiment, it is assumed that the feature vector $g_f(x)$ for the input medical data is generated every individual finding. The feature vector for the input medical data may be expressed as a real number vector having a random natural number c dimension.

The first diagnosis network may include an individual finding weight vector $w_f$ and an individual finding bias value $b_f$ for each finding in order to generate the first diagnosis information including at least finding information based on the input medical data. The first diagnosis network transposes the individual finding weight vector

$$w_f \in R^C \quad (w_f^T)$$

and multiplies the feature vector for the input medical data by the individual finding weight vector and adds the individual finding bias value $b_f \in R$ and inputs the added individual finding bias value into the activation function $\sigma$ to calculate the existence probability of at least one finding included in the first diagnosis information. The first diagnosis network may include a final classification function based on the activation function. The individual finding weight vector $w_f$ and the individual finding bias value $b_f$ may be parameters used for the first diagnosis network to drive the existence probability of the specific finding f from the feature vector. The individual finding weight vector $w_f$ and the individual finding bias value $b_f$ may unique parameters which exist every finding. The parameters may be updated or trained through learning.

As in the above example, the processor 110 may train each individual finding weight vector $w_f$ included in the first diagnosis network and the individual finding bias value $b_f$ included in the first diagnosis network in order to output the first diagnosis information including at least one finding information based on the input medical data. Further, during the resulting backpropagation process, the processor 110 may train and update the internal parameter in the first diagnosis network in order to calculate the feature vector $g_f(x)$ for the input medical data with respect to the input data x. In the example of the training method of the first diagnosis network, the type of activation function is just an example, and does not limit the present disclosure.

According to an embodiment of the present disclosure, the processor 110 may train the deep learning based first diagnosis network as described above, and then fix an internal parameter value included in the trained first diagnosis network and train the second diagnosis network for D predetermined (or selected) diseases. The processor 110 may calculate a feature vector for at least one input medical data by inputting medical data labeled with information on one or more diseases, i.e., the second learning data set into the deep learning based first diagnosis network. The processor 110 may obtain the second diagnosis information including at least one disease information by inputting the feature vector for the calculated input medical data into the second diagnosis network. As a specific example, a specific equation of an objective function trained for the second diagnosis network to output the second diagnosis information is shown in Equation 2.

[Equation 2]

$$\hat{y}_d(x) = \sigma\left(\sum_{f=f_1}^{f_F} v_{d,f}^T g_f(x) + c_d\right) \quad (f \in \{f_1, f_2, \ldots, f_F\}, d \in \{1, 2, \ldots, D\})$$

x represents the input medical data and $\hat{y}_d(x)$ represents a diagnosis probability of a specific disease d included in the second diagnosis information which is the output of the input medical data of the second diagnosis network. d as an element of a set comprised of D predetermined (or selected) diseases represents the individual disease. f represents at least one individual finding. $g_f(x) \in R^C$ represents a feature vector for the input medical data calculated in the first diagnosis network after the processor 110 inputs the input medical data x into the first diagnosis network. The feature vector $g_f(x)$ for the input medical data may be a feature vector for calculating the existence probability of each of two or more findings. $v_{d,f}$ represents a disease-finding weight vector. $c_d$ represents a bias value depending on the disease. $\sigma$ represents the activation function.

The second diagnosis network may include a disease (d)-finding (f) weight vector $v_{d,f}$ every all disease (d)-finding (f) pairs and a bias value $c_d$ depending on the disease every all diseases in order to generate the second diagnosis information based on a feature vector for input medical data related to at least one finding.

The second diagnosis network may multiply the feature vector $g_f(x)$ for the input medical data by the disease-finding weight vector $v_{d,f} \in R^C$ for each finding $f \in \{f_1, f_2, \ldots, f_F\}$. The second diagnosis network transposes the disease-finding weight vector $v_{d,f} \in R^C$ ($v_{d,f}^T$) and multiplies the feature vector $g_f(x)$ for the input medical data by the disease-finding weight vector according to each finding $f \in \{f_1, f_2, ..., f_F\}$ and then sums up values obtained by multiplication ($\Sigma$), and adds the bias value $c_d \in R$ depending on the corresponding disease, and inputs the added bias value into the activation function $\sigma$ to output the second diagnosis information. The second diagnosis network may include the final classification function based on the activation function. The second diagnosis information may include a diagnosis probability of at least one disease d. The disease-finding weight vector $v_{d,f}$ and the bias value $c_d$ depending on the disease may be parameters used for the second diagnosis network to derive a final diagnosis result by receiving the feature vector $g_f(x)$ for the input medical data extracted from initial input medical data by the first diagnosis network again. The disease-finding weight vector $v_{d,f}$ may be a unique parameter which exists every disease-finding pair. The bias value $c_d$ depending on the disease may be a unique parameter which exists every disease. The parameters may be updated or trained through learning. The processor 110 may fix the internal parameter of the first diagnosis network model by obtaining the trained first diagnosis network, input the initial medical data into the first diagnosis network and train the second diagnosis network based on the feature vector $g_f(x)$ for the input medical data calculated from the first diagnosis network.

As described above, according to the present disclosure in which when training the second diagnosis network, the processor 110 first trains the first diagnosis network and extracts the feature vector for the input medical data in the first diagnosis network which is pretrained, and trains the second diagnosis network based thereon, there are following advantages. First, the feature vector for the input medical data is expressed as an array having a dimension, and as a result, position information of the lesion required for expressing the finding is not lost. In other words, since only whether there is a finding which exists in initially input medical data may be checked through the feature vector and the location of the finding which exits, the region of the finding, and ground data through which the first diagnosis network determines the finding are maintained as internal parameter values, when all internal parameter values are provided to the second diagnosis network, a large amount of information may be transferred without a loss rather than just transferring the final probability of the finding existence. Therefore, since the second diagnosis network is provided with high-quality input data in inferring a relation between the disease and the finding, inference performance is enhanced. Second, the first diagnosis network and the second diagnosis network share the feature vector for the input medical data, and as a result, the processor 110 may calculate a contribution of at least one finding to the disease diagnosed through the output of the second diagnosis network. This has an effect in that an entire medical diagnosis model is capable of presenting the determination ground of the diagnosis for the disease rather than just generating only the second diagnosis information for the disease.

In an embodiment of the present disclosure, the processor 110 may output (or generate) correlation information including the first diagnosis information and the second diagnosis information calculated for the input medical data based on a part of a calculation process of the first diagnosis network or a calculation process of the second diagnosis network. The correlation information may include first diagnosis information calculated for the input medical data, second diagnosis information calculated for the input medical data, contributions of one or more findings included in the first diagnosis information to at least one disease included in the second diagnosis information, data obtained by visualizing the contributions, an odds ratio, an odds ratio matrix, a class activation map displaying the first diagnosis information or the second diagnosis information on the input medical data, etc.

In an embodiment of the present disclosure, the correlation information including the first diagnosis information and the second diagnosis information may include the contributions of one or more finding information included in the first diagnosis information to at least one disease included in the second diagnosis information. The processor 110 calculates the contributions to quantify an influence exerted by the first diagnosis information including the existence probability of the individual finding when a specific disease included in the second diagnosis information is output from the input medical data. That is, the first diagnosis information or the finding information which becomes the ground of the second diagnosis information may be just provided and degrees of the influences may be provided as numerical values so as to compare sizes of influences which existences of one or more findings included in the first diagnosis information exerts on the second diagnosis information.

The processor 110 may calculate the contributions of one or more finding information included in the first diagnosis information to at least one disease included in the second diagnosis information based on at least any one of the feature vector for the input medical data calculated in the first diagnosis network, the parameter of the final classification function included in the first diagnosis network or the parameter of the final classification function included in the second diagnosis network. The final classification function included in the first diagnosis network or the final classification function included in the second diagnosis network includes a linear classification function in which an inverse function exists, such as a sigmoid function, a Leaky ReLU function, etc., without a limit. The parameter of the final classification function included in the first diagnosis network may include the individual finding weight vector $w_f$ and the individual finding bias value $b_f$ included in the first diagnosis network. The parameter of the final classification function included in the second diagnosis network may include the disease ($d$)-finding ($f$) weight vector $v_{d,f}$ and the bias value $c_d$ depending on the disease. Since the processor 110 calculates the contribution of one or more finding information included in the first diagnosis information to at least one disease included in the second diagnosis information based on at least some of the parameter values of the linear classification function which exists in the trained first diagnosis network or second diagnosis network, the processor 110 does not additionally require a separate learning data set or a separate network function for calculating the contribution. Further, as disclosed in the present disclosure, when the contribution is calculated, the first diagnosis information and the second diagnosis information may be output from the input medical data and correlation information between both diagnosis information may be known.

Hereinafter, a method for calculating the contribution of one or more finding information included in the first diagnosis information to at least one disease included in the second diagnosis information will be described.

According to an embodiment of the present disclosure, the processor 110 may calculate the contribution of one or more finding information included in the first diagnosis information to at least one disease included in the second diagnosis information based on odds. In this specification, the odds may be interchanged as a meaning shown in Equation 3, and used.

[Equation 3]

$$odds = \frac{P}{1 - P}$$

In Equation 3 above, P may mean a probability that a specific event will occur. That is, through this specification, the odds may mean "a radio of a probability that an event will occur and a probability that the event will occur". For example, when a probability that the artificial neural network will determine an image obtained by photographing eyeballs through ophthalmic equipment as glaucoma is 80%, a probability that the image will not be determined as the glaucoma is 20%, and as a result, odds of input data for diagnosis information for the glaucoma may be expressed as 0.8 / (1 - 0.8) = 0.8 / 0.2 = 4.

In an embodiment of the present disclosure, the processor 110 may calculate the contribution of one or more finding included in the first diagnosis information to at least one disease included in the second diagnosis information based on at least one of a first partial contribution or a second partial contribution. The first partial contribution may mean a value indicating an influence by all findings included in the first diagnosis information for at least one disease included in the second diagnosis information. The second partial contribution may mean a value indicating an influence by one or more findings included in the first diagnosis information for at least one disease included in the second diagnosis information.

The processor 110 may calculate the first partial contribution based on odds of all findings included in the first diagnosis information for at least one disease included in the second diagnosis information. In the present disclosure, the odds of all findings included in the first diagnosis information for at least one disease included in the second diagnosis information may be used as the same meaning as odds in which a specific disease d will exist in the input medical data. The first partial contribution may be expressed as a ratio of a probability that the specific disease d will exist and a probability that the specific disease d will not exist in the input medical data. In the present disclosure, a method for calculating the first partial contribution by the processor 110 as an example may be based on Equation 4 below.

[Equation 4]

$$O(d; x) = \frac{\hat{y}_d(x)}{1 - \hat{y}_d(x)} = exp\left(\sum_{f=f_1}^{f_F} v_{d,f}^T g_f(x) + c_d\right)$$

As such, the first partial contribution calculated by the processor 110 may be expressed as odds $O(d; x)$ of input medical data x for information on the disease d. That is, the processor 110 may calculate the first partial contribution based on second diagnosis information including an existence probability $\hat{y}_d(x)$ for at least one disease d, which is output by a medical diagnosis model including the trained first diagnosis network and the trained second diagnosis network. In addition, a right formula of a last equal sign in Equation 4 may be derived from Equations 5 and 6 when the sigmoid function $\sigma(x)$ is used as the activation function.

[Equation 5]

$$\hat{y}_d(x) = \sigma\left(\sum_{f=f_1}^{f_F} v_{d,f}^T g_f(x) + c_d\right)$$

[Equation 6]

$$\frac{\sigma(x)}{1 - \sigma(x)} = e^x$$

Therefore, referring to Equations 4 to 6, the processor 110 may input medical data into the medical diagnosis model including the trained first diagnosis network and the trained second diagnosis network, and calculate the first partial contribution based on at least one of a feature vector $g_f(x)$ for input medical data related to at least one finding f extracted from the first diagnosis network, a disease-finding weight vector $v_{d,f}$ as the parameter of the final classification function included in the second diagnosis network, and a bias value $c_d$ depending on the disease as the parameter of the final classification function included in the second diagnosis network.

In addition to the operation of inferring the existence of the disease for the medical data input without a separate external device or an additional input as described above, the processor 110 may additionally perform the operation of calculating the first partial contribution which is based on the odds in which the disease will exist in the input medical data based on a calculation using internal parameter values.

Next, a method for calculating the second partial contribution by the processor 110 will be described. The second partial contribution may mean a value indicating an influence by one or more findings included in the first diagnosis information for at least one disease included in the diagnosis information. In an embodiment of the present disclosure, the processor 110 may calculate the second partial contribution based on counterfactual-odds of one or more findings included in the first diagnosis information for at least one disease included in the second diagnosis information.

In this specification, the counterfactual-odds of one or more findings included in the first diagnosis information for at least one disease included in the second diagnosis information may be interchanged and used as the same meaning as odds according to counterfactual existence probabilities of one or more findings included in the first diagnosis information for at least one disease included in the second diagnosis information. The counterfactual-odds may mean odds according to a counterfactual existence probability of each finding included in the first diagnosis information contributing to the calculation of the second diagnosis information when the second diagnosis information including a specific type of disease is calculated from the input data.

For example, when Diabetic Retinopathy (DR) is diagnosed as the second diagnosis information for the input medical data, such second diagnosis information diagnosed by the processor 110 may be based on first diagnosis information including existence probabilities for a plurality of findings including Hemorrhage (Hmr) and Hard Exudate (HE). In this case, the counterfactual-odds of one or more finding information included in the first diagnosis information (Hemorrhage (Hmr) and Hard Exudate (HE)) for the second diagnosis information (Diabetic Retinopathy (DR)) may include counterfactual-odds assuming a situation in which the finding of Hemorrhage (Hmr) exists 100%, counterfactual-odds assuming a situation in which the finding of Hemorrhage (Hmr) exists 0%, and counterfactual-odds assuming a situation in which the finding of Hemorrhage (Hmr) exists predetermined (or selected) p%. Further, the counterfactual-odds of one or more finding information included in the first diagnosis information (Hemorrhage (Hmr) and Hard Exudate (HE)) for the second diagnosis information (Diabetic Retinopathy (DR)) may include counterfactual-odds assuming a situation in which the finding of Hemorrhage (Hmr) exists 100% and the finding of Hard Exudate (HE) exists 0%. That is, in the present disclosure, the counterfactual-odds of one or more findings included in the first diagnosis information for at least one disease included in the second diagnosis information may mean odds in which when the processor 110 calculates the second diagnosis information from the input data, the existence probability of at least one included in the first diagnosis information which becomes a basis of the calculation is suppositively set, so the resulting second diagnosis information will be derived.

Hereinafter, a method for calculating the counterfactual-odds of one or more findings included in the first diagnosis information for at least one disease included in the second diagnosis information will be described. Hereinafter, as an example of the present disclosure, a method for adjusting the existence probability for one finding included in the first diagnosis information and calculating the resulting counterfactual-odds will be described, but this is just an example, and the present disclosure includes a method for adjusting the existence probabilities of a plurality of findings included in the first diagnosis information for the second diagnosis information and calculating the resulting counterfactual-odds without a limit.

The processor 110 may adjust an influence of a specific finding in the feature vector for the input medical data calculated in the first diagnosis network in order to calculate the counterfactual-odds. The processor 110 may adjust the influence of the specific finding in the feature vector for the input medical data based on a calculation of dismantling the feature vector into two-direction different vectors. The processor 110 dismantles the feature vector into a first vector in a direction parallel to the individual finding weight vector $w_f$ and a second vector in the remaining direction to adjust the influence of the specific finding in the feature vector for the input medical data. The remaining-direction second vector may include a vector in a direction orthogonal to the individual finding weight vector $w_f$. A process in which the processor 110 dismantles the feature vector into two different directions may be performed based on Equation 7.

[Equation 7]

$$g_f(x) = \frac{w_f^T\, g_f(x)}{\left\|w_f\right\|}w_f + \left[g_f(x) - \frac{w_f^T\, g_f(x)}{\left\|w_f\right\|}w_f\right] = \frac{\sigma^{-1}\!\left(\hat{y}_f(x)\right) - b_f}{\left\|w_f\right\|}w_f + g_{f\perp w_f}(x)$$

In Equation 7, a right formula of a first equal sign indicates that the feature vector $g_f(x)$ for the input medical data is dismantled into a first vector $\dfrac{w_f^T\, g_f(x)}{\left\|w_f\right\|}w_f$ in a direction parallel to the individual finding weight vector $w_f$ and a second vector $\left[g_f(x) - \dfrac{w_f^T\, g_f(x)}{\left\|w_f\right\|}w_f\right]$ in the remaining direction. In Equation 7 above, a first term of a right formula of a last equal sign may be derived by taking an inverse function $\sigma^{-1}$ of the sigmoid function used as the activation function in Equation 1 described above on both sides, and shifting each term. That is, in Equation 7, the first term of the right formula of the last equal sign may mean the first vector in the direction parallel to the individual finding weight vector $w_f$ in the feature vector $g_f(x)$. The first vector parallel to parallel to the individual finding weight vector $w_f$ may be calculated based on the feature vector derived by the network function and the existence probability $\hat{y}_f(x)$ of the individual finding included in the output first diagnosis information after the processor 110 inputs the input medical data into the first diagnosis network. In addition, in Equation 7, a second term $g_{f\perp w_f}(x)$ of the right formula of the last equal sign may mean the remaining-direction second vector in the feature vector. The second vector may include a component vector orthogonal to the individual finding weight vector $w_f$. The second vector may be calculated based on a difference between the feature vector $g_f(x)$ and the first vector.

According to an embodiment of the present disclosure, when the processor 110 adjusts the existence probabilities of the plurality of findings for the second diagnosis information and calculates the resulting counterfactual-odds, the processor 110 may dismantle the feature vector in a plurality of different directions. Specifically, in order to adjust existence probabilities of two findings expressed as $f_1$ and $f_2$, and calculate the resulting counterfactual-odds, the processor 110 may dismantle the feature vector $g_f(x)$ for the input medical data into three directions. Specifically, the processor 110 may dismantle the feature vector $g_f(x)$ for the input medical data into a third vector in a direction parallel to $f_1$, a fourth vector in a direction parallel to $f_2$, and a fifth vector in the remaining direction. The third vector may be calculated as in $\dfrac{w_{f_1}^T\, g_f(x)}{\left\|w_{f_1}\right\|}w_{f_1}$ and the fourth vector may be calculated as in $\dfrac{w_{f_2}^T\, g_f(x)}{\left\|w_{f_2}\right\|}w_{f_2}$. The remaining fifth vector may be calculated by differences between the feature vector $g_f(x)$, and the third vector and the fourth vector.

The processor 110 changes the existence probability $\hat{y}_f(x)$ of the finding f included in the first diagnosis information based on the above-described feature vector dismantling method to calculate the counterfactual-odds of one or more findings included in the first diagnosis information for at least one disease included in the second diagnosis information. The processor 110 dismantles the feature vector $g_f(x)$ for the input medical data extracted from the first diagnosis network and then substitutes another probability value p for the existence probability $\hat{y}_f(x)$ of the specific finding included in the first diagnosis information which is the output of the first diagnosis network to calculate odds of the second diagnosis information according to the changed probability value. The processor 110 may remove or adjust the influence of the specific finding in the feature vector. For example, a feature vector $g_{-f}(x)$ in which the existence probability of the specific finding f is substituted with 0 to remove the influence of the finding may be expressed as Equation 8.

[Equation 8]

$$g_{-f}(x) = \frac{\sigma^{-1}(0) - b_f}{\left\|w_f\right\|} w_f + g_{f \perp w_f}(x)$$

That is, the processor 110 may remove the influence of the specific finding from the feature vector based on dismantling the feature vector into two different directions and then substituting the existence probability $\hat{y}_f(x)$ of the specific finding input into the inverse function $\sigma^{-1}$ of the activation function with 0 which is another probability value p. As another embodiment, when a probability value p of 1 is substituted for the existence probability $\hat{y}_f(x)$ of the specific finding input into the inverse function $\sigma^{-1}$ of the activation function, a feature vector depending on an existence probability of a situation in which the specific finding particularly exists may be obtained. Furthermore, when the existence probability $\hat{y}_f(x)$ of the specific finding input into the inverse function $\sigma^{-1}$ of the activation function is set to a random predetermined (or selected) probability value p, the feature vector depending on the existence probability of the finding may be obtained. As such, the influence of the specific finding from the feature vector is adjusted by a calculation using the network internal functions and parameters of the processor 110.

Hereinafter, a method for calculating the counterfactual-odds of one or more findings included in the first diagnosis information for at least one disease included in the second diagnosis information from the feature vector depending on each existence probability of the specific finding will be described.

Throughout this specification, the counterfactual-odds of one or more findings included in the first diagnosis information for at least one disease included in the second diagnosis information may be interchanged and referred to as the same meaning as odds $C(d, f, p; x)$ according to the counterfactual existence probability p of the individual finding f included in the first diagnosis information for the second diagnosis information including information on the specific disease d for the input medical data x. The processor 110 may use the counterfactual-odds for calculation of the second partial contribution which becomes the basis of the contribution calculation. The counterfactual existence probability p of the individual finding may be 0 or 1 and may be a random value between 0 and 1, such as 0.5, 0.7, etc. The counterfactual-odds of one or more findings included in the first diagnosis information for one disease included in the second diagnosis information which becomes a basis for the processor 110 to calculate the second partial contribution may include at least one of odds depending on a probability (p = 1) of a situation in which one or more findings included in the first diagnosis information for at least one disease included in the second diagnosis information particularly exists, odds depending on a probability (p = 0) of a situation in which one or more findings included in the first diagnosis information for at least one disease included in the second diagnosis information do not absolutely exist, or odds depending on predetermined (or selected) existence probabilities (0 < $p$ < 1) of one or more findings included in the first diagnosis information for one disease included in the second diagnosis information. The first diagnosis information may include counterfactual existence probabilities for a plurality of findings. The second partial contribution when the influence of the specific finding is removed, in other words, the odds $C(d, f, p; x)$ depending on the counterfactual existence probability p of the individual finding f included in the first diagnosis information for the second diagnosis information including the information on the specific disease d for the input medical data x may be calculated as in Equation 9.

[Equation 9]

$$C(d, f, 0; x) = exp\left(\sum_{i \neq f} v_{d,i}^T g_i(x) + v_{d,f}^T g_{-f}(x) + c_d\right)$$

Equation 9 may be described with reference to Equation 4 described above. Equation 9 may be derived by sequentially adding values according to respective findings with respect to the $v_{d,i}^T g_i(x)$ term when $i \neq f$ and adding a term $v_{d,f}^T g_{-f}(x)$ from which the influence of the finding is removed only when i is a specific finding ($i = f$). The counterfactual-odds $C(d, f, 0; x)$ calculated by the processor 110 according to an embodiment of the present disclosure as described above may include a ratio of a probability that the specific disease d will be diagnosed and a probability that it will be diagnosed that the specific disease will not exist when the influence of the specific finding f is removed from the input medical data x by an inter-vector calculation ($p = 0$). An example of the above-described probability value ($p = 0$) is just an example and the counterfactual-odds may be calculated according to the predetermined (or selected) existence probability of the individual finding.

The processor 110 may use trained parameters included in the first diagnosis network or the second diagnosis network in the process of outputting the first diagnosis information and the second diagnosis information based on the first diagnosis network or the second diagnosis network including at least one network function. The parameters included in the first diagnosis network or the second diagnosis network may include at least one of the vector $g_f(x)$ for the input medical data, the individual finding weight vector $w_f$, the individual finding bias value $b_f$, or the disease-finding weight vector $v_{d,f}$. The processor 110 calculates the counterfactual-odds of one or more findings included in the first diagnosis information for at least one disease included in the second diagnosis information by using the trained parameters to quantify influences by one or more findings included in the first diagnosis information for the second diagnosis information. This enables a user to interpret on which finding the artificial neural network reaches such a conclusion based in addition to a case where the medical diagnosis model just provides the disease existence or the disease existence probability.

The processor 110 may express the contribution of one or more findings f included in the first diagnosis information to at least one disease d included in the second diagnosis information based on an odds ratio. In this specification, the odds ratio may be used as the same meaning as a cross ratio or a multiplication ratio. The odds ratio may mean a ratio of two odds derived under an assumption that two odds are different. The odds as a basis of the calculation of the odds ratio may be odds of all findings included in the first diagnosis information for at least one disease included in the second diagnosis information. The odds as the basis of the calculation of the odds ratio may be counterfactual odds of one or more findings included in the first diagnosis information for at least one disease included in the second diagnosis information.

In this specification, "the odds ratio for the influence which the specific finding f included in the first diagnosis information exerts on the second diagnosis information for the specific disease d" and "the counterfactual-odds-ratio" may be interchanged and used as the same meaning.

In the present disclosure, the processor 110 may express the contribution of one or more findings included in the first diagnosis information to at least one disease included in the second diagnosis information as a ratio of a first partial contribution and a second partial contribution. As a specific example, an equation for the counterfactual odds ratio ($p = 0$) may be expressed as Equation 10.

[Equation 10]

$$R_{CO}(d, f, 0; x) = \frac{O(d; x)}{C(d, f, 0; x)} = exp\left( v_{d,f}^T \, w_f \left( \frac{\sigma^{-1}(\hat{y}_f) - \sigma^{-1}(0)}{\left\| w_f \right\|} \right) \right)$$

Equation 10 showing the counterfactual-odds ratio may be have a value larger than 1 when there is a positive correlation between the specific finding and the disease. Specifically, when the medical diagnosis model determines that the disease d exists in the input medical data, if the processor 110 removes the influence of the specific finding f which exerts a meaningful influence on diagnosing that the corresponding disease exists, a probability that the disease will occur decreases, and as a result, a value of the counterfactual odds $C(d, f, 0; x)$ becomes smaller and a denominator of the counterfactual-odds ratio $R_{CO}(d, f, 0; x)$ becomes smaller. Therefore, it may be meant that as the value of the counterfactual-odds ratio $R_{CO}(d, f, 0; x)$ meaning an influence of the specific finding f on the disease d becomes larger, a degree at which the finding f which exists in the input medical data x contributes to generating the second diagnosis information for the corresponding disease d by the second diagnosis network is larger.

The contributions of one or more findings included in the first diagnosis information to at least one disease included in the second diagnosis information calculated by the processor 110 and included in the correlation information may include discovery importance $R_{DF}(d, f)$ of one or more findings f included in the first diagnosis information for one or more diseases d included in the second diagnosis information. The processor 110 may be at least partially based on the second partial contribution in order to calculate the discovery importance $R_{DF}(d, f)$. The discovery importance $R_{DF}(d, f)$ may be calculated based on the odds $C(d, f, 1; x)$ depending on the probability of the situation in which the specific finding f included in the first diagnosis information for one or more diseases d included in the second diagnosis information particularly exists. The discovery importance $R_{DF}(d, f)$ may be calculated based on the odds $C(d, f, 0; x)$ depending on the probability of the situation in which the specific finding f included in the first diagnosis information for one or more diseases d included in the second diagnosis information does not absolutely exist. As an example, the discovery importance may be calculated based on the ratio between the two odds $C(d, f, 1; x)$ and $C(d, f, 0; x)$. The formula is shown in Equation 11 below.

[Equation 11]

$$R_{DF}(d, f) = \frac{C(d, f, 1; x)}{C(d, f, 0; x)} = exp\left( v_{d,f}^T \, w_f \left( \frac{\sigma^{-1}(1) - \sigma^{-1}(0)}{\|w_f\|} \right) \right)$$

As shown in Equation 11, the processor 110 calculates the discovery importance $R_{DF}(d, f)$ of one or more findings f included in the first diagnosis information for one or more diseases d included in the second diagnosis information based on the second partial contribution to quantify an importance on which discovering the corresponding finding in the input medical data exerts on diagnosing the disease in a relation with another finding.

The above-described formulas use the sigmoid function as the activation function in order to describe the contribution calculating scheme in the present disclosure. However, the activation function includes all activation functions capable of taking the inverse function, which include a tanh function, a Leaky ReLU function, and the like without a limit. Furthermore, the substituted probability value may also be changed into a random predetermined (or selected) value, and calculated without a limit. That is, the above-described example does not limit the present disclosure, and it will be apparent to those skilled in the art that an object and an effect to be achieved by the present disclosure may be achieved even through a change of a simple value or a change of the function.

In general, the artificial neural network derives a final output through a calculation process between multitudinous nodes for the initial input data. Accordingly, even though the output may be obtained through the artificial neural network, the user may not easily know which information of the input data the artificial neural network such a result is derived based. On the contrary, the processor 110 of the present disclosure may quantitatively derive the influence of the first diagnosis information on the second diagnosis information through the correlation information between the first diagnosis information and the second diagnosis information which are finally output. Through this, an independent variable is controlled in a causal relation in which multiple independent variables are involved to determine dependent variables to identify a correlation between the independent variable and the dependent variable. As a result, the present disclosure may provide a high-transparency and high-reliability result of a deep learning based network function. That is, in industrial fields including a medical field, etc., when a ground for the final determination of the artificial neural network is submitted and a high reliability is required, there is an effect that the reliability of the artificial neural network may be quantitatively provided.

According to an embodiment of the present disclosure, when the processor 110 outputs the correlation information including the first diagnosis information and the second diagnosis information, the processor 110 may visualize and provide the contributions of one or more findings included in the first diagnosis information to at least one disease included in the second diagnosis information. The processor 110 may output second diagnosis information including existence of the disease or an existence probability of the disease for the input medical data, first diagnosis information including one or more finding information contributing to the corresponding second diagnosis information, or the contribution of the first diagnosis information as values. The processor 110 may visualize and provide the values for readability of the user. The visualization operation may include an operation of representing a difference in contribution between findings with a difference in size between expression factors according to a graph by expressing a contribution distribution of each first diagnosis information with the graph, an operation of displaying a lesion region of a finding having a large contribution in the input data, or an operation of giving a difference in darkness of the color in relation to a contribution of the finding information included in the first diagnosis information to the second diagnosis information according to a degree of the contribution and expressing the contribution in a 2D matrix.

The processor 110 may display and output the first diagnosis information in the input medical data based on at least a part of a class activation map in outputting the correlation information including the first diagnosis information and the second diagnosis information. The processor 110 may express by which pixel region of the input medical data the second diagnosis information which is a final diagnosis result of the medical diagnosis model is output through the class activation map. The processor 110 may generate the class activation map based on the feature vector $g_f(x)$ for the input medical data. In an embodiment of the present disclosure, when a feature vector $g_f(x) \in R^{C'}$ is extracted by the global average pooling method, the processor 110 may generate the class activation map based on a feature map $\dot{g}_f(x) \in R^{C' \times H' \times W'}$ just before the feature vector $g_f(x)$ is subjected to global average pooling. The formula is shown in

Equation 12.

[Equation 12]

$$A(d,f|x) = \frac{v_{d,f}^T \, w_f}{\left\| w_f \right\|} \left( w_f^T \dot{g}_f(x) \right) \; \left( A(d,f|x) \in R^{H' \times W'} \right)$$

The processor 110 may generate the class activation map at least partially based on a disease-finding weight vector $v_{d,f}$ which is a parameter by which the feature vector for the input medical data is multiplied for each finding in order for the second diagnosis network to generate the second diagnosis information. Specifically, the processor 110 may determine a weight value by which each channel of a feature map $\dot{g}_f(x)$ is multiplied based on the disease-finding weight vector $v_{d,f}$ in order to generate the class activation map.

According to an example of the present disclosure, the processor 110 may generate the class activation map based at least any one of the feature vector for the input medical data, the parameter of the final classification function included in the first diagnosis network, or the parameter of the final classification function included in the second diagnosis network, and display and output the first diagnosis information in the input medical data based on the class activation map, in outputting the correlation information including the first diagnosis information and the second diagnosis information. The processor 110 may obtain a feature map $\dot{g}_f(x)$ which is data just before the feature vector $g_f(x)$ for the input medical data is extracted by the global average pooling method during the calculation process of the first diagnosis network in a calculation result of the first diagnosis network. As shown in Equation 12 above, the processor 110 multiplies the feature map $\dot{g}_f(x)$ by a component vector in a direction parallel to a vector of the individual finding weight $w_f$ included in the final classification function of the first diagnosis network and a disease-finding weight vector $v_{d,f}$ included in the final classification function of the second diagnosis network for each channel, and sums up values obtained through the multiplication to generate the class activation map based thereon. The processor 110 may display a lesion region related to one or more finding information included in the first diagnosis information which influences the output of the second diagnosis information in the input medical data through the class activation map.

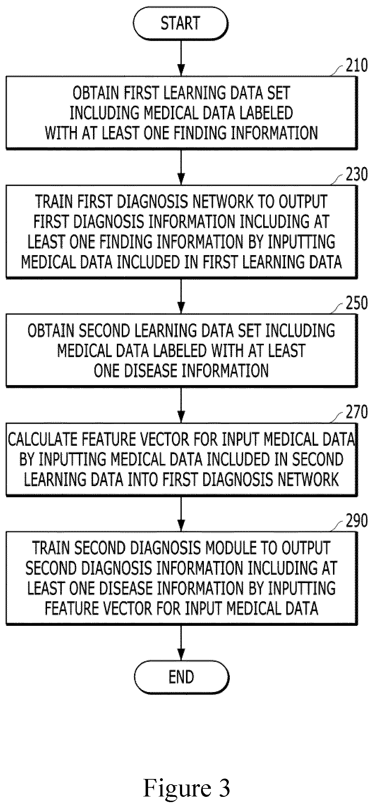

Hereinafter, a training process will be described with reference to Figure 3. Figure 3 is a flowchart for a training process of a global model for outputting first diagnosis information and second diagnosis information from input medical data according to an embodiment of the present disclosure providing diagnostic related information for medical data. The processor 110 may obtain a first learning data set including medical data labeled with at least one finding information (210). The first diagnosis information may include the existence of the finding, the location of the finding, the probability for the finding, and the like for the medical data. The processor 110 may train the first diagnosis network to output at least one finding information by inputting medical data included in first learning data (230). The processor 110 may obtain a second learning data set including medical data labeled with at least one disease information (250). In this case, a step of obtaining the second learning data set (250) and a step of training the first diagnosis network (230) may also be changed with each other in an order. The processor 110 may calculate the feature vector for the input medical data by inputting the medical data included in the second learning data into the first diagnosis network (270). The feature vector for the input medical data may include data calculated on an intermediate calculation process for the first diagnosis network to output the first diagnosis information for the input medical data. The processor 110 may train the second diagnosis network to output the second diagnosis information including at least one disease information by inputting the feature vector for the input medical data. The second diagnosis information may include a name of the disease, existence of the disease, a ground finding of the disease, a lesion region of the ground finding, and the like. The second diagnosis network may be comprised of a network function and comprised of a decision tree in order to calculate the second diagnosis information from the feature vector for the input medical data. The processor 110 may calculate the contribution of the first diagnosis information to one or more second diagnosis information output by inputting the medical data into the medical diagnosis model including the trained first diagnosis network and the trained second diagnosis network.

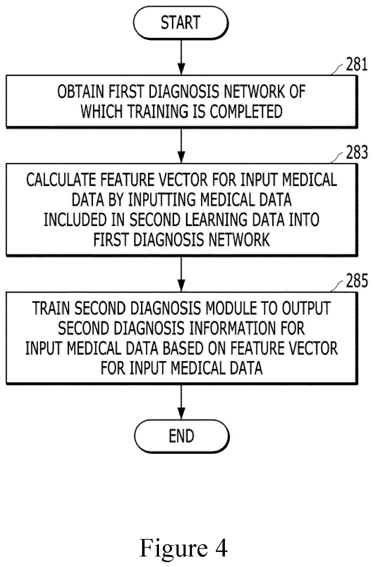

Figure 4 is a flowchart for training a second diagnosis network according to an embodiment of the present disclosure providing diagnostic related information for medical data. The processor 110 may obtain a deep learning based first diagnosis network of which training is completed (281). A case where the training is completed includes a meaning that parameter values included in the deep learning based first diagnosis network are not trained or updated any longer. After fixing a model of the deep learning based first diagnosis network, the processor 110 may calculate a feature vector for at least one input medical data by inputting medical data included in the second learning data into the first diagnosis network (283). Thereafter, the processor 110 may train the second diagnosis network to output the second diagnosis information for the medical data based on the feature vector for the input medical data (285). A case of training the second diagnosis network based on the feature vector on an internal calculation process of the first diagnosis network other than a final classification result of the first diagnosis network means a case where a vector type containing more information than a numeral value including only a simple value is provided as the learning data of the second diagnosis network. Accordingly, the second diagnosis network may more accurately learn and infer the correlation between the second diagnosis information and the first diagnosis information.

As described above, the processor 110 sequentially trains the first diagnosis network and the second diagnosis network included in a global medical diagnosis model separately stepwise, and as a result, the second diagnosis network may be trained based on vector type intermediate data having a predetermined (or selected) specific dimension calculated by the first diagnosis network. If the first diagnosis network and the second diagnosis network are simultaneously trained, a combination of the first diagnosis network and the second diagnosis network is not different from one single network as a whole. Further, when the first diagnosis network and the second diagnosis network are simultaneously trained, the feature vector for the input medical data is continuously repeatedly modified and updated during a training process, and as a result, the second diagnosis network may not be provided with the feature vector for the input medical data calculated from the first diagnosis network as the learning data. Accordingly, when all networks are trained like one network, a relation between the feature vector for the input medical data and the second diagnosis information may not be learned, and as result, the correlation information between the second diagnosis information and the first diagnosis information may not be output. On the contrary, in the present disclosure, after the first diagnosis network is first trained, the second diagnosis network is trained by using the calculation result of the first diagnosis network to train the medical diagnosis model considering an influence on the specific disease of the individual finding. In other words, the finding which contributes to a specific diagnosis may be quantitatively identified.

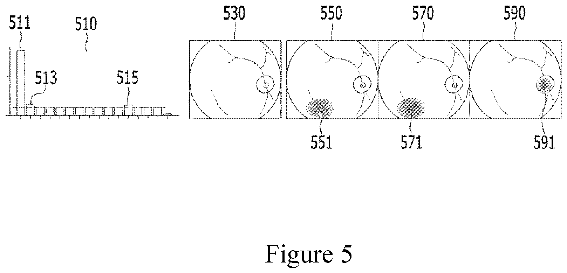

Figure 5 is a diagram for visualizing correlation information including first diagnosis information and second diagnosis information for input medical data according to an embodiment of the present disclosure for providing diagnostic related information for medical data. A bar graph 510 of Figure 5 is a graph that expresses the first diagnosis information which contributes to the second diagnosis information according to the contribution size. In the embodiment of Figure 5, the second diagnosis information may be second diagnosis information including only information on one disease. A size of a bar for one or more findings included in the first diagnosis information may be in proportion to a ratio of a degree at which each finding influences the disease. The processor 110 may select a first finding 511 having a largest contribution, a second finding 513 having a second largest contribution, and a third finding 515 a third largest contribution among all findings according to the contribution size for the disease for each finding.

The processor 110 may display first diagnosis information including information on one or more findings in the input medical data based on the class activation map. The processor 110 may generate first medical data 550 displaying a lesion 551 for the first finding, second medical data 570 displaying a lesion 571 for the second finding, and third medical data 590 displaying a lesion 591 for the third finding with respect to original medical data 530. Three findings described above may be findings comprised of three higher-level findings according to the contribution size calculated by the processor 110 in the first diagnosis information including all findings. The number of findings selected by the processor 110 is just an example, and does not limit the present disclosure. Since the processor 110 may output the contribution size of the finding included in the first diagnosis information for the specific disease through the bar graph 510, the processor 110 may transfer a cause of a diagnosis result to the user at a glance, and further, display the lesion region for each finding in the original medical data 530 as described above to provide data which enables rapidly determining the location of the finding to the user.

According to an embodiment of the present disclosure, disclosed is a computer readable recording medium storing a data structure storing data related to a calculation of a neural network providing diagnostic related information for the medical data. The data related to the calculation of the neural network providing the diagnostic related information for the medical data may be obtained by the following operation of the processor 110. The processor 110 may train the first diagnosis network based on the first learning data set including medical data labeled with at least one finding information, train the second diagnosis network at least partially based on the feature vector for the medical data included in the second learning data set calculated by inputting the second learning data set including medical data labeled with at least one disease information into the first diagnosis network, and calculate correlation information between a first diagnosis information set for the first learning data set and a second diagnosis information set included in the second learning data set at least partially based on a learning result of the first diagnosis network and a learning result of the second diagnosis network. That is, the processor 110 may infer correlation information between the first diagnosis information, the second diagnosis information, or each diagnosis information for new medical data by training the first diagnosis network and the second diagnosis network, and visualize and output a correlation between all second diagnosis information and all first diagnosis information calculated from the learning data set through parameter values obtained during a training process.

According to the embodiment of the present disclosure, a computing device 100 may further include a display unit (not illustrated). The display unit may include at least one of a liquid crystal display (LCD), a thin film transistor-liquid crystal display (TFT LCD), an organic light-emitting diode (OLED), a flexible display, and a 3D display. Some display modules among them may be configured as a transparent or light transmissive type to view the outside through the displays. This may be called a transparent display module and a representative example of the transparent display module includes a transparent OLED (TOLED), and the like. The type of display unit described above is just an example, but the computing device according to the present disclosure may include all types of display devices for a display.

The processor 110 may display correlation information including first diagnosis information and second diagnosis information for input medical data through the display unit. The correlation information may include first diagnosis information calculated for the input medical data, second diagnosis information calculated for the input medical data, a contribution of the first diagnosis information to the second diagnosis information, data obtained by visualizing the contributions, an odds ratio, an odds ratio matrix, a class activation map displaying the first diagnosis information or the second diagnosis information on the input medical data, etc.

The processor 110 may display the correlation information as an odds ratio matrix including the contribution of the first diagnosis information to the second diagnosis information in displaying the correlation information including the first diagnosis information and the second diagnosis information.

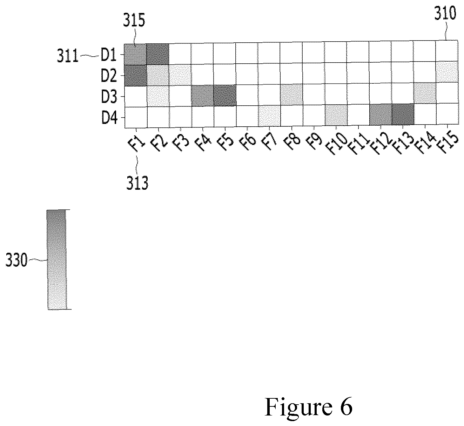

According to an embodiment of the present disclosure, the processor 110 of the computing device 100 may transmit, to a user terminal, correlation information including first diagnosis information for input medical data and second diagnosis information for the input medical data through the network unit 150. The user terminal may include at least one of components included in the computing device illustrated in Figure 1. The user terminal may receive the correlation information including the first diagnosis information and the second diagnosis information from an external server by using a network unit or receive the medical data according to the above-described methods by using the processor included in the user terminal and generate the correlation information including the first diagnosis information and the second diagnosis information. The user terminal may provide a user interface that display diagnostic related information for medical data. The user terminal may provide a user interface including the correlation information including the first diagnostic information and the second diagnostic information through an output unit. The user terminal may display a user interface including at least a part of a screen configuration illustrated in Figure 6. Hereinafter, a method for displaying the correlation information based on the odds ratio matrix will be described with reference to Figure 6.

Figure 6 is a diagram for a user interface displaying correlation information including first diagnosis information and second diagnosis information from input medical data according to an embodiment of the present disclosure for providing diagnostic related information for medical data. A network function trained by the processor 110 may display a correlation between the first diagnosis information and the second diagnosis information based on parameter values included in the network function. Individual gratings included in an odds ratio matrix 310 of Figure 6 may individually represent contribution sizes of one or more findings included in the first diagnosis information for each of at least one disease included in the second diagnosis information. The individual gratings included in the odds ratio matrix 310 may express the contribution size of each finding based on the above-described counterfactual-odds ratio. The odds ratio expression may be an expression of a color for distinguishing an absolute size difference or a relative size difference. The processor 110 may express contributions of individual findings which influence on a specific disease in the odds ratio matrix 310 with different colors according to sizes of the contributions. For example, in respect to a disease 311 called D1 included in the second diagnosis information output for the input medical data, gratings of individual findings included in the first diagnosis information may be expressed with different colors according to the contribution sizes. In Figure 6, it may be known through a color in a grating 315 for a D1 - F1 pair that a contribution of finding F1 313 for disease D1 is larger than those of the remaining findings other than finding F2. A vertical axis of the odds ratio matrix 310 may include at least one disease included in the second diagnosis information. A horizontal axis of the odds ratio matrix 310 may include one or more findings included in the first diagnosis information. A color bar 330 of Figure 6 may indicate a color corresponding to a distribution of a minimum value and a maximum value of the contribution calculated by the processor 110. The minimum value and the maximum value of the color bar 330 may be extracted from a global contribution distribution or selected as a random value in advance.

The data structure may refer to the organization, management, and storage of data that enables efficient access to and modification of data. The data structure may refer to the organization of data for solving a specific problem (e.g., data search, data storage, data modification in the shortest time). The data structures may be defined as physical or logical relationships between data elements, designed to support specific data processing functions. The logical relationship between data elements may include a connection relationship between data elements that the user thinks. The physical relationship between data elements may include an actual relationship between data elements physically stored on a computer-readable storage medium (e.g., hard disk). The data structure may include may specifically include a set of data, a relation between the data, a function which may be applied to the data, or instructions. Through an effectively designed data structure, a computing device can perform operations while using the resources of the computing device to a minimum. Specifically, the computing device can increase the efficiency of operation, read, insert, delete, compare, exchange, and search through the effectively designed data structure.

The data structure may be divided into a linear data structure and a non-linear data structure according to the type of data structure. The linear data structure may be a structure in which only one data is connected after one data. The linear data structure may include a list, a stack, a queue, and a deque. The list may mean a series of data sets in which an order exists internally. The list may include a linked list. The linked list may be a data structure in which data is connected in a manner that each data is connected in a row with a pointer. In the connection list, the pointer may include connection information with next or previous data. The linked list may be represented as a single linked list, a double linked list, or a circular linked list depending on the type. The stack may be a data listing structure with limited access to data. The stack may be a linear data structure that may process (e.g., insert or delete) data at only one end of the data structure. The data stored in the stack may be a data structure (LIFO-Last in First Out) in which the data is input last and output first. The queue is a data arrangement structure that may access data limitedly and unlike a stack, the queue may be a data structure (FIFO-First in First Out) in which late stored data is output late. The deck may be a data structure capable of processing data at both ends of the data structure.

The nonlinear data structure may be a structure in which a plurality of data are connected after one data. The non-linear data structure may include a graph data structure. The graph data structure may be defined as a vertex and an edge, and the edge may include a line connecting two different vertices. The graph data structure may include a tree data structure. The tree data structure may be a data structure in which there is one path connecting two different vertices among a plurality of vertices included in the tree. That is, the tree data structure may be a data structure that does not form a loop in the graph data structure.

Throughout the present specification, a computation model, the neural network, a network function, and the neural network may be used as the same meaning. (Hereinafter, the computation model, the neural network, the network function, and the neural network will be integrated and described as the neural network). The data structure may include the neural network. In addition, the data structures, including the neural network, may be stored in a computer readable medium. The data structure including the neural network may also include data input to the neural network, weights of the neural network, hyper parameters of the neural network, data obtained from the neural network, an active function associated with each node or layer of the neural network, and a loss function for training the neural network. The data structure including the neural network may include predetermined (or selected) components of the components disclosed above. In other words, the data structure including the neural network may include all of data input to the neural network, weights of the neural network, hyper parameters of the neural network, data obtained from the neural network, an active function associated with each node or layer of the neural network, and a loss function for training the neural network or a combination thereof. In addition to the above-described configurations, the data structure including the neural network may include predetermined (or selected) other information that determines the characteristics of the neural network. In addition, the data structure may include all types of data used or generated in the calculation process of the neural network, and is not limited to the above. The computer readable medium may include a computer readable recording medium and/or a computer readable transmission medium. The neural network may be generally constituted by an aggregate of calculation units which are mutually connected to each other, which may be called nodes. The nodes may also be called neurons. The neural network is configured to include one or more nodes.

The data structure may include data input into the neural network. The data structure including the data input into the neural network may be stored in the computer readable medium. The data input to the neural network may include training data input in a neural network training process and/or input data input to a neural network in which training is completed. The data input to the neural network may include preprocessed data and/or data to be preprocessed. The preprocessing may include a data processing process for inputting data into the neural network. Therefore, the data structure may include data to be preprocessed and data generated by preprocessing. The data structure is just an example and the present disclosure is not limited thereto.

The data structure may include data input into the neural network or data output from the neural network. The data structure including the data input into or output from the neural network may be stored in the computer readable medium. The data structure stored in the computer readable medium may include data input in a neural network inference process or output data output as a result of the neural network inference. In addition, the data structure may include data processed by a specific data processing method, and thus may include data before and after processing. Therefore, the data structure may include data to be processed and data processed through a data processing method.

The data structure may include weights of the neural network (weights and parameters may be used as the same meaning in the present disclosure). In addition, the data structures, including the weight of the neural network, may be stored in the computer readable medium. The neural network may include a plurality of weights. The weight may be variable and the weight is variable by a user or an algorithm in order for the neural network to perform a desired function. For example, when one or more input nodes are mutually connected to one output node by the respective links, the output node may determine an output node value based on values input in the input nodes connected with the output node and the parameters set in the links corresponding to the respective input nodes. The data structure is just an example and the present disclosure is not limited thereto.

As a non-limiting example, the weight may include a weight which varies in the neural network training process and/or a weight in which neural network training is completed. The weight which varies in the neural network training process may include a weight at a time when a training cycle starts and/or a weight that varies during the training cycle. The weight in which the neural network training is completed may include a weight in which the training cycle is completed. Accordingly, the data structure including the weight of the neural network may include a data structure including the weight which varies in the neural network training process and/or the weight in which neural network training is completed. Accordingly, the above-described weight and/or a combination of each weight are included in a data structure including a weight of a neural network. The data structure is just an example and the present disclosure is not limited thereto.

The data structure including the weight of the neural network may be stored in the computer-readable storage medium (e.g., memory, hard disk) after a serialization process. Serialization may be a process of storing data structures on the same or different computing devices and later reconfiguring the data structure and converting the data structure to a form that may be used. The computing device may serialize the data structure to send and receive data over the network. The data structure including the weight of the serialized neural network may be reconstructed in the same computing device or another computing device through deserialization. The data structure including the weight of the neural network is not limited to the serialization. Furthermore, the data structure including the weight of the neural network may include a data structure (for example, B-Tree, Trie, m-way search tree, AVL tree, and Red-Black Tree in a nonlinear data structure) to increase the efficiency of operation while using resources of the computing device to a minimum. The above-described matter is just an example and the present disclosure is not limited thereto.

The data structure may include hyper-parameters of the neural network. In addition, the data structures, including the hyper-parameters of the neural network, may be stored in the computer readable medium. The hyper-parameter may be a variable which may be varied by the user. The hyper-parameter may include, for example, a learning rate, a cost function, the number of learning cycle iterations, weight initialization (for example, setting a range of weight values to be subjected to weight initialization), and Hidden Unit number (e.g., the number of hidden layers and the number of nodes in the hidden layer). The data structure is just an example and the present disclosure is not limited thereto.

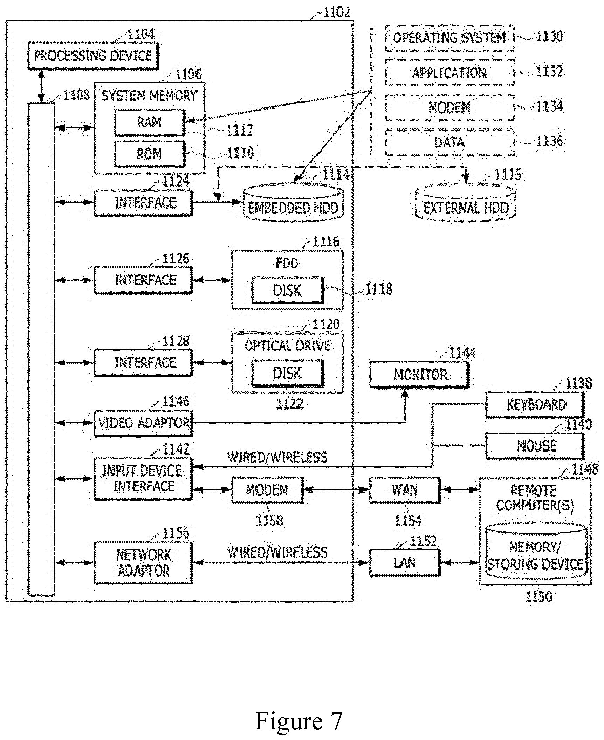

Figure 7 is a simple and normal schematic view of a computing environment in which the embodiments of the present disclosure may be implemented.

It is described above that the present disclosure may be generally implemented by the computing device, but those skilled in the art will well know that the present disclosure may be implemented in association with a computer executable command which may be executed on one or more computers and/or in combination with other program modules and/or as a combination of hardware and software.

In general, the program module includes a routine, a program, a component, a data structure, and the like that execute a specific task or implement a specific abstract data type. Further, it will be well appreciated by those skilled in the art that the method of the present disclosure can be implemented by other computer system configurations including a personal computer, a handheld computing device, microprocessor-based or programmable home appliances, and others (the respective devices may operate in connection with one or more associated devices as well as a single-processor or multi-processor computer system, a mini computer, and a main frame computer.

The embodiments described in the present disclosure may also be implemented in a distributed computing environment in which predetermined (or selected) tasks are performed by remote processing devices connected through a communication network. In the distributed computing environment, the program module may be positioned in both local and remote memory storage devices.

The computer generally includes various computer readable media. Media accessible by the computer may be computer readable media regardless of types thereof and the computer readable media include volatile and non-volatile media, transitory and non-transitory media, and mobile and non-mobile media. As a non-limiting example, the computer readable media may include both computer readable storage media and computer readable transmission media. The computer readable storage media include volatile and non-volatile media, temporary and non-temporary media, and movable and non-movable media implemented by a predetermined (or selected) method or technology for storing information such as a computer readable instruction, a data structure, a program module, or other data. The computer readable storage media include a RAM, a ROM, an EEPROM, a flash memory or other memory technologies, a CD-ROM, a digital video disk (DVD) or other optical disk storage devices, a magnetic cassette, a magnetic tape, a magnetic disk storage device or other magnetic storage devices or predetermined (or selected) other media which may be accessed by the computer or may be used to store desired information, but are not limited thereto.

The computer readable transmission media generally implement the computer readable command, the data structure, the program module, or other data in a carrier wave or a modulated data signal such as other transport mechanism and include all information transfer media. The term "modulated data signal" means a signal acquired by configuring or changing at least one of characteristics of the signal so as to encode information in the signal. As a non-limiting example, the computer readable transmission media include wired media such as a wired network or a direct-wired connection and wireless media such as acoustic, RF, infrared and other wireless media. A combination of any media among the aforementioned media is also included in a range of the computer readable transmission media.

An environment 1100 that implements various aspects of the present disclosure including a computer 1102 is shown and the computer 1102 includes a processing device 1104, a system memory 1106, and a system bus 1108. The system bus 1108 connects system components including the system memory 1106 (not limited thereto) to the processing device 1104. The processing device 1104 may be a predetermined (or selected) processor among various commercial processors. A dual processor and other multi-processor architectures may also be used as the processing device 1104.

The system bus 1108 may be any one of several types of bus structures which may be additionally interconnected to a local bus using any one of a memory bus, a peripheral device bus, and various commercial bus architectures. The system memory 1106 includes a read only memory (ROM) 1110 and a random access memory (RAM) 1112. A basic input/output system (BIOS) is stored in the non-volatile memories 1110 including the ROM, the EPROM, the EEPROM, and the like and the BIOS includes a basic routine that assists in transmitting information among components in the computer 1102 at a time such as in-starting. The RAM 1112 may also include a high-speed RAM including a static RAM for caching data, and the like.

The computer 1102 also includes an interior hard disk drive (HDD) 1114 (for example, EIDE and SATA), in which the interior hard disk drive 1114 may also be configured for an exterior purpose in an appropriate chassis (not illustrated), a magnetic floppy disk drive (FDD) 1116 (for example, for reading from or writing in a mobile diskette 1118), and an optical disk drive 1120 (for example, for reading a CD-ROM disk 1122 or reading from or writing in other high-capacity optical media such as the DVD, and the like). The hard disk drive 1114, the magnetic disk drive 1116, and the optical disk drive 1120 may be connected to the system bus 1108 by a hard disk drive interface 1124, a magnetic disk drive interface 1126, and an optical drive interface 1128, respectively. An interface 1124 for implementing an exterior drive includes at least one of a universal serial bus (USB) and an IEEE 1394 interface technology or both of them.

The drives and the computer readable media associated therewith provide non-volatile storage of the data, the data structure, the computer executable instruction, and others. In the case of the computer 1102, the drives and the media correspond to storing of predetermined (or selected) data in an appropriate digital format. In the description of the computer readable media, the mobile optical media such as the HDD, the mobile magnetic disk, and the CD or the DVD are mentioned, but it will be well appreciated by those skilled in the art that other types of media readable by the computer such as a zip drive, a magnetic cassette, a flash memory card, a cartridge, and others may also be used in an operating environment and further, the predetermined (or selected) media may include computer executable commands for executing the methods of the present disclosure.

Multiple program modules including an operating system 1130, one or more application programs 1132, other program module 1134, and program data 1136 may be stored in the drive and the RAM 1112. All or some of the operating system, the application, the module, and/or the data may also be cached in the RAM 1112. It will be well appreciated that the present disclosure may be implemented in operating systems which are commercially usable or a combination of the operating systems.

A user may input instructions and information in the computer 1102 through one or more wired/wireless input devices, for example, pointing devices such as a keyboard 1138 and a mouse 1140. Other input devices (not illustrated) may include a microphone, an IR remote controller, a joystick, a game pad, a stylus pen, a touch screen, and others. These and other input devices are often connected to the processing device 1104 through an input device interface 1142 connected to the system bus 1108, but may be connected by other interfaces including a parallel port, an IEEE 1394 serial port, a game port, a USB port, an IR interface, and others.

A monitor 1144 or other types of display devices are also connected to the system bus 1108 through interfaces such as a video adapter 1146, and the like. In addition to the monitor 1144, the computer generally includes other peripheral output devices (not illustrated) such as a speaker, a printer, others.

The computer 1102 may operate in a networked environment by using a logical connection to one or more remote computers including remote computer(s) 1148 through wired and/or wireless communication. The remote computer(s) 1148 may be a workstation, a computing device computer, a router, a personal computer, a portable computer, a micro-processor based entertainment apparatus, a peer device, or other general network nodes and generally includes multiple components or all of the components described with respect to the computer 1102, but only a memory storage device 1150 is illustrated for brief description. The illustrated logical connection includes a wired/wireless connection to a local area network (LAN) 1152 and/or a larger network, for example, a wide area network (WAN) 1154. The LAN and WAN networking environments are general environments in offices and companies and facilitate an enterprise-wide computer network such as Intranet, and all of them may be connected to a worldwide computer network, for example, the Internet.

When the computer 1102 is used in the LAN networking environment, the computer 1102 is connected to a local network 1152 through a wired and/or wireless communication network interface or an adapter 1156. The adapter 1156 may facilitate the wired or wireless communication to the LAN 1152 and the LAN 1152 also includes a wireless access point installed therein in order to communicate with the wireless adapter 1156. When the computer 1102 is used in the WAN networking environment, the computer 1102 may include a modem 1158 or has other means that configure communication through the WAN 1154 such as connection to a communication computing device on the WAN 1154 or connection through the Internet. The modem 1158 which may be an internal or external and wired or wireless device is connected to the system bus 1108 through the serial port interface 1142. In the networked environment, the program modules described with respect to the computer 1102 or some thereof may be stored in the remote memory/storage device 1150. It will be well known that an illustrated network connection is and other means configuring a communication link among computers may be used.

The computer 1102 performs an operation of communicating with predetermined (or selected) wireless devices or entities which are disposed and operated by the wireless communication, for example, the printer, a scanner, a desktop and/or a portable computer, a portable data assistant (PDA), a communication satellite, predetermined (or selected) equipment or place associated with a wireless detectable tag, and a telephone. This at least includes wireless fidelity (Wi-Fi) and Bluetooth wireless technology. Accordingly, communication may be a predefined structure like the network in the related art or just ad hoc communication between at least two devices.

The wireless fidelity (Wi-Fi) enables connection to the Internet, and the like without a wired cable. The Wi-Fi is a wireless technology such as the device, for example, a cellular phone which enables the computer to transmit and receive data indoors or outdoors, that is, anywhere in a communication range of a base station. The Wi-Fi network uses a wireless technology called IEEE 802.11(a, b, g, and others) in order to provide safe, reliable, and high-speed wireless connection. The Wi-Fi may be used to connect the computers to each other or the Internet and the wired network (using IEEE 802.3 or Ethernet). The Wi-Fi network may operate, for example, at a data rate of 11 Mbps (802.11a) or 54 Mbps (802.11b) in unlicensed 2.4 and 5 GHz wireless bands or operate in a product including both bands (dual bands).

It will be appreciated by those skilled in the art that information and signals may be expressed by using various different predetermined (or selected) technologies and techniques. For example, data, instructions, commands, information, signals, bits, symbols, and chips which may be referred in the above description may be expressed by voltages, currents, electromagnetic waves, magnetic fields or particles, optical fields or particles, or predetermined (or selected) combinations thereof.

It may be appreciated by those skilled in the art that various logical blocks, modules, processors, means, circuits, and algorithm steps described in association with the embodiments disclosed herein may be implemented by electronic hardware, various types of programs or design codes (for easy description, herein, designated as software), or a combination of all of them. In order to clearly describe the intercompatibility of the hardware and the software, various components, blocks, modules, circuits, and steps have been generally described above in association with functions thereof. Whether the functions are implemented as the hardware or software depends on design restrictions given to a specific application and an entire system. Those skilled in the art of the present disclosure may implement functions described by various methods with respect to each specific application, but it should not be interpreted that the implementation determination departs from the scope of the present disclosure.

Various embodiments presented herein may be implemented as manufactured articles using a method, an apparatus, or a standard programming and/or engineering technique. The term manufactured article includes a computer program, a carrier, or a medium which is accessible by a predetermined (or selected) computer-readable storage device. For example, a computer-readable storage medium includes a magnetic storage device (for example, a hard disk, a floppy disk, a magnetic strip, or the like), an optical disk (for example, a CD, a DVD, or the like), a smart card, and a flash memory device (for example, an EEPROM, a card, a stick, a key drive, or the like), but is not limited thereto. Further, various storage media presented herein include one or more devices and/or other machine-readable media for storing information.

It will be appreciated that a specific order or a hierarchical structure of steps in the presented processes is one example of accesses. It will be appreciated that the specific order or the hierarchical structure of the steps in the processes within the scope of the present disclosure may be rearranged based on design priorities. Appended method claims provide elements of various steps in a sample order, but the method claims are not limited to the presented specific order or hierarchical structure.

The description of the presented embodiments is provided so that those skilled in the art of the present disclosure use or implement the present disclosure. Various modifications of the embodiments will be apparent to those skilled in the art and general principles defined herein can be applied to other embodiments without departing from the scope of the present disclosure. Therefore, the present disclosure is not limited to the embodiments presented herein, but should be interpreted within the widest range which is coherent with the principles and new features presented herein.

## Claims

1. A method of providing diagnostic related information for medical data, performed by one or more processors of a computing device, the method comprising:

   calculating first diagnosis information on input medical data by using a first diagnosis network trained to output diagnosis information based on the medical data;
   calculating second diagnosis information on the input medical data by using a second diagnosis network trained to output diagnosis information based on a feature vector for the medical data calculated in the first diagnosis network, wherein the feature vector is calculated based on a feature map derived from a calculation process of the first diagnosis network; and
generating correlation information comprising the first diagnosis information and the second diagnosis information calculated on the input medical data, based on a part of a calculation process of the first diagnosis network or a calculation process of the second diagnosis network.

2. The method of claim 1, wherein the calculating first diagnosis information on input medical data by using the first diagnosis network further comprising:

calculating a feature vector for the input medical data in the first diagnosis network; and
calculating the first diagnosis information based on the feature vector for the input medical data.

3. The method of claim 1, wherein the feature vector is calculated based on a result of performing a global pooling method on the feature map derived from the calculation process of the first diagnosis network.

4. The method of claim 1, wherein the first diagnosis network comprises two or more different sub first diagnosis networks for calculating first diagnosis information comprising different types of findings.

5. The method of claim 1, wherein the correlation information comprises a contribution of one or more findings comprised in the first diagnosis information to at least one disease comprised in the second diagnosis information.

6. The method of claim 5, wherein the contribution is calculated based on at least one of a feature vector for the input medical data calculated in the first diagnosis network, a parameter of a final classification function comprised in the first diagnosis network, or a parameter of a final classification function comprised in the second diagnosis network.

7. The method of claim 5, wherein the contribution is calculated based on at least one of a first partial contribution or a second partial contribution.

8. The method of claim 7, wherein the first partial contribution is based on odds of all findings comprised in first diagnosis information for at least one disease comprised in the second diagnosis information.

9. The method of claim 7, wherein the second partial contribution is based on counterfactual-odds of one or more findings comprised in the first diagnosis information for at least one disease comprised in the second diagnosis information, and
wherein the counterfactual-odds comprises at least one of an odd according to a probability of a situation in which one or more findings comprised in the first diagnosis information for at least one disease comprised in the second diagnosis information necessarily exist, an odd according to a probability of a situation in which one or more findings comprised in the first diagnosis information for at least one disease comprised in the second diagnosis information never exist, or an odd according to a predetermined probability of existence of one or more findings comprised in the first diagnosis information for at least one disease comprised in the second diagnosis information.

10. The method of claim 1, wherein the generating correlation information comprising:
displaying the first diagnosis information in the input medical data based at least in part on a class activation map.

11. The method of claim 1, wherein the generating correlation information comprising:

generating a class activation map based on at least one of a feature vector of the input medical data, a parameter of a final classification function comprised in the first diagnosis network, or a parameter of a final classification function comprised in the second diagnosis network; and
displaying the first diagnosis information in the input medical data based on the class activation map.

12. A computing device for providing diagnostic related information for medical data, comprising:

a processor; and
a memory in which at least one network function is stored,
wherein the memory stores at least one computer-executable instruction for the processor to:

calculate first diagnosis information on input medical data by using a first diagnosis network trained to output diagnosis information based on medical data;

calculate second diagnosis information on the input medical data by using a second diagnosis network trained to output diagnosis information based on a feature vector for the medical data calculated in the first diagnosis network, wherein the feature vector is calculated based on a feature map derived from a calculation process of the first diagnosis network; and

generate correlation information comprising the first diagnosis information and the second diagnosis information calculated on the input medical data, based on a part of a calculation process of the first diagnosis network or a calculation process of the second diagnosis network.

13. A computer program stored in a computer readable storage medium wherein when the computer program is executed in one or more processors comprised in a user terminal, the computer program provides a user interface(UI) for displaying diagnostic related information for medical data, the user interface comprising:

correlation information comprising first diagnosis information of input medical data and second diagnosis information of the input medical data; and

wherein the correlation information is generated based on a part of a calculation process of first diagnosis information using a first diagnosis network and a calculation process of second diagnosis information using a second diagnosis network, and which is generated from a user terminal or a server.

14. The computer program stored in a computer readable storage medium of claim 13, wherein the correlation information comprising:

an odds ratio matrix comprising a contribution of one or more findings comprised in the first diagnosis information to at least one disease comprised in the second diagnosis information.

100

PROCESSOR — 110

MEMORY — 130

NETWORK UNIT — 150

Figure 1

Figure 2

START

210

OBTAIN FIRST LEARNING DATA SET
INCLUDING MEDICAL DATA LABELED
WITH AT LEAST ONE FINDING INFORMATION

230

TRAIN FIRST DIAGNOSIS NETWORK TO OUTPUT
FIRST DIAGNOSIS INFORMATION INCLUDING AT
LEAST ONE FINDING INFORMATION BY INPUTTING
MEDICAL DATA INCLUDED IN FIRST LEARNING DATA

250

OBTAIN SECOND LEARNING DATA SET INCLUDING
MEDICAL DATA LABELED WITH AT LEAST
ONE DISEASE INFORMATION

270

CALCULATE FEATURE VECTOR FOR INPUT MEDICAL DATA
BY INPUTTING MEDICAL DATA INCLUDED IN SECOND
LEARNING DATA INTO FIRST DIAGNOSIS NETWORK

290

TRAIN SECOND DIAGNOSIS MODULE TO OUTPUT
SECOND DIAGNOSIS INFORMATION INCLUDING AT
LEAST ONE DISEASE INFORMATION BY INPUTTING
FEATURE VECTOR FOR INPUT MEDICAL DATA

END

Figure 3

```
                    ┌─────────────┐
                    │    START    │
                    └──────┬──────┘
                           │                          ╱ 281
         ┌─────────────────▼──────────────────┐
         │   OBTAIN FIRST DIAGNOSIS NETWORK OF │
         │     WHICH TRAINING IS COMPLETED     │
         └─────────────────┬──────────────────┘
                           │                          ╱ 283
         ┌─────────────────▼──────────────────┐
         │  CALCULATE FEATURE VECTOR FOR INPUT MEDICAL │
         │        DATA BY INPUTTING MEDICAL DATA       │
         │      INCLUDED IN SECOND LEARNING DATA INTO   │
         │           FIRST DIAGNOSIS NETWORK           │
         └─────────────────┬──────────────────┘
                           │                          ╱ 285
         ┌─────────────────▼──────────────────┐
         │   TRAIN SECOND DIAGNOSIS MODULE TO OUTPUT   │
         │       SECOND DIAGNOSIS INFORMATION FOR      │
         │  INPUT MEDICAL DATA BASED ON FEATURE VECTOR │
         │           FOR INPUT MEDICAL DATA            │
         └─────────────────┬──────────────────┘
                           │
                    ┌──────▼──────┐
                    │     END     │
                    └─────────────┘
```

Figure 4

Figure 5

Figure 6

Figure 7

## INTERNATIONAL SEARCH REPORT

| | International application No. |
|---|---|
| | **PCT/KR2021/007853** |

### A. CLASSIFICATION OF SUBJECT MATTER

**G16H 15/00**(2018.01)i; **G16H 40/20**(2018.01)i; **G16H 10/60**(2018.01)i; **G16H 70/00**(2018.01)i; **G16H 80/00**(2018.01)i; **G06N 20/00**(2019.01)i; **G06N 3/08**(2006.01)i

According to International Patent Classification (IPC) or to both national classification and IPC

### B. FIELDS SEARCHED

Minimum documentation searched (classification system followed by classification symbols)

G16H 15/00(2018.01); A61B 5/00(2006.01); A61B 5/055(2006.01); G06N 3/02(2006.01); G06T 5/00(2006.01); G06T 7/00(2006.01); G06T 7/11(2017.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 의료 데이터(medical data), 진단 정보(diagnosis information), 진단 네트워크 (diagnosis network)

### C. DOCUMENTS CONSIDERED TO BE RELEVANT

| Category* | Citation of document, with indication, where appropriate, of the relevant passages | Relevant to claim No. |
|---|---|---|
| X | KR 10-2018-0076504 A (AJOU UNIVERSITY INDUSTRY-ACADEMIC COOPERATION FOUNDATION) 06 July 2018 (2018-07-06)<br>See paragraphs [0024], [0035]-[0036] and [0065]; claims 1, 3 and 7; and figures 2-3. | 1-2,5-9,12-14 |
| Y | | 3-4,10-11 |
| Y | KR 10-1889725 B1 (LUNIT INC.) 20 August 2018 (2018-08-20)<br>See paragraphs [0024] and [0157]-[0158]; and figure 15. | 3-4,10-11 |
| A | KR 10-2018-0021635 A (KOREA ADVANCED INSTITUTE OF SCIENCE AND TECHNOLOGY) 05 March 2018 (2018-03-05)<br>See paragraphs [0035]-[0061]; and figure 1. | 1-14 |
| A | KR 10-2020-0020079 A (KOREA ADVANCED INSTITUTE OF SCIENCE AND TECHNOLOGY) 26 February 2020 (2020-02-26)<br>See paragraphs [0077]-[0078]; and figure 5. | 1-14 |

☑ Further documents are listed in the continuation of Box C.   ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **14 October 2021** | **14 October 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
|---|
| **PCT/KR2021/007853** |

**C.     DOCUMENTS CONSIDERED TO BE RELEVANT**

| Category* | Citation of document, with indication, where appropriate, of the relevant passages | Relevant to claim No. |
|---|---|---|
| A | KR 10-2015-0098119 A (SAMSUNG ELECTRONICS CO., LTD.) 27 August 2015 (2015-08-27)<br>See paragraphs [0062]-[0082]; and figures 8-10. | 1-14 |

Form PCT/ISA/210 (second sheet) (July 2019)

**INTERNATIONAL SEARCH REPORT**
Information on patent family members

| International application No. |
| --- |
| **PCT/KR2021/007853** |

| Patent document cited in search report | | | Publication date (day/month/year) | Patent family member(s) | | | Publication date (day/month/year) |
| --- | --- | --- | --- | --- | --- | --- | --- |
| KR | 10-2018-0076504 | A | 06 July 2018 | KR | 10-1876338 | B1 | 09 July 2018 |
| KR | 10-1889725 | B1 | 20 August 2018 | KR | 10-1889722 | B1 | 20 August 2018 |
| | | | | KR | 10-1889723 | B1 | 20 August 2018 |
| | | | | KR | 10-1889724 | B1 | 20 August 2018 |
| KR | 10-2018-0021635 | A | 05 March 2018 | KR | 10-1980955 | B1 | 21 May 2019 |
| KR | 10-2020-0020079 | A | 26 February 2020 | KR | 10-2183847 | B1 | 27 November 2020 |
| KR | 10-2015-0098119 | A | 27 August 2015 | CN | 104840209 | A | 19 August 2015 |
| | | | | EP | 2911111 | A2 | 26 August 2015 |
| | | | | EP | 2911111 | A3 | 23 September 2015 |
| | | | | JP | 2015-154918 | A | 27 August 2015 |
| | | | | US | 2015-0230773 | A1 | 20 August 2015 |
| | | | | US | 9532762 | B2 | 03 January 2017 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020180123198